# EUROPEAN PATENT APPLICATION

(11) **EP 1 932 854 A1**
(43) Date of publication of application: **18.06.2008**
(21) Application number: 06291948.5
(22) Date of filing: 15.12.2006
(51) Int. Cl.: C07K 14/47, C07K 14/705, C07K 16/30, C12N 15/12, G01N 33/50

(54) **Lysosomal associated membrane protein (LAMP) - like polypeptide, ligands to the same, and use of the same in the frame of detection and purification of human plasmacytoid dendritic cells**

(71) Applicant: INSTITUT NATIONAL DE LA SANTE ET DE LA RECHERCHE MEDICALE (INSERM), 75013 Paris (FR)
(72) Inventor: Pierre, Philippe, 13010 Marseille (FR); David, Alexandre, 13300 Salon de Provence (FR)
(74) Representative: Colombet, Alain André

(57) **Abstract**

The present invention relates to an isolated protein comprising or consisting of:
- a sequence represented by SEQ ID NO: 2 (*Homo sapiens* BAD-LAMP), or
- a homologous sequence which presents at least 20% identity to SEQ ID NO: 2, provided that the homologous sequence represents a protein having an activity similar to that of *Homo sapiens* BAD-LAMP.

## Description

### Field of the invention

The present invention relates to a previously unknown protein, sharing significant homology with Lysosomal Associated Membrane Proteins (LAMPs), to ligands specific for this protein, and to the use this protein for detecting or purifying human dendritic plasmacytoid dendritic cells.

### Background of the invention

LAMPs form a family of heavily glycosylated membrane glycoproteins which are mostly localized in lysosomes and have been shown to play an important role in their biogenesis (Eskelinen et al. (2003) Trends Cell Biol 13:137).

Plasmacytoid dendritic cells are a subset of dendritic cells which are substantially absent in normal skin and which have been shown to occur in cutaneous inflammatory diseases, such as poriasis vulgaris, contact dermatitis, and lupus erythematosus (Wollenberg et al. (2002) J. Invest Dermatol. 119:1096; Bangert et al. (2003) J. Invest Dermatol. 121:1409). They have been suggested to be useful in the frame of cancer treatment (Palamera et al. (2004) J. Immunol. 173:3051) as well as in the treatment of infectious diseases, such as viral infections, due to their type I interferon secretion capacities (Siegal et al. (1999) Science 284:1835).

Plasmacytoid dendritic cells were originally characterized as medium-sized cells having a plasma cell-like morphology (Lennert & Remmele (1958) Acta Hematol. 19:99). However, they are devoid of the typical markers of plasma cells and are mostly localized in T cell zones of lymphoid tissues. The expression of several markers, such as CD123, CD45RA, CD68, CXCR3 and CD62L, was shown to be associated with these cells. Furthermore it was also shown that these cells express CD4 and MHC class II molecules but not CD11c and CD45RO (Palamera et al. (2004) J. Immunol. 173:3051). In general, plasmacytoid dendritic cells are characterized as being CD11c⁻ CD123⁺ cells. Furthermore, BDCA-2 and BDCA-4 have been found to be relatively specific markers of plasmacytoid dendritic cells (Dzionek et al. (2000) J. Immunol. 165:6037).

As such, there is a need for alternative, more specific biochemical markers of plasmacytoid dendritic cells.

### Summary of the Invention

The present invention arises from the finding, by the present inventors, of a previously unknown protein sharing a certain homology with LAMPs, which they named BAD-LAMP, and from the finding that, in man, among non-neuronal cells, the expression of the gene encoding this protein was restricted to a subset of dendritic cells known as plasmacytoid dendritic cells.

Thus, the present invention relates to an isolated protein comprising or consisting of:
- a sequence represented by SEQ ID NO: 2 (*Homo sapiens* BAD-LAMP), or
- a homologous sequence which presents at least 20% identity to SEQ ID NO: 2, preferably at least 40%, more preferably at least 60%, most preferably at least 80%, provided that the homologous sequence represents a protein having an activity similar to that of *Homo sapiens* BAD-LAMP.

In an embodiment, the above-defined isolated protein is represented by a sequence selected from the group consisting of SEQ ID NO: 2, SEQ ID NO: 4 (*Mus musculus* BAD-LAMP), SEQ ID NO: 6 (*Rattus norvegicus* BAD-LAMP), SEQ ID NO: 8 *(Bos taurus* BAD-LAMP), SEQ ID NO: 10 *(Pan troglodytes* BAD-LAMP), SEQ ID NO: 12 *(Monodelphis domestica* BAD-LAMP), SEQ ID NO: 14 *(Xenopus tropicalis* BAD-LAMP), SEQ ID NO: 16 *(Gallus gallus* BAD-LAMP), SEQ ID NO: 18 (*Anopheles gambiae* BAD-LAMP), SEQ ID NO: 20 (*Tetraodon viridis* BAD-LAMP), SEQ ID NO: *22* (*Takifugu rubripes* BAD-LAMP) and SEQ ID NO: 24 (*Drosophila melanogaster* BAD-LAMP).

In a preferred embodiment, the above-defined isolated protein is represented by a sequence selected from the group consisting of SEQ ID NO: 2, SEQ ID NO: 4, SEQ ID NO: 6, SEQ ID NO: 8, and SEQ ID NO: 10.

In another preferred embodiment the above-defined isolated protein is represented by SEQ ID NO: 2 or SEQ ID NO: 4.

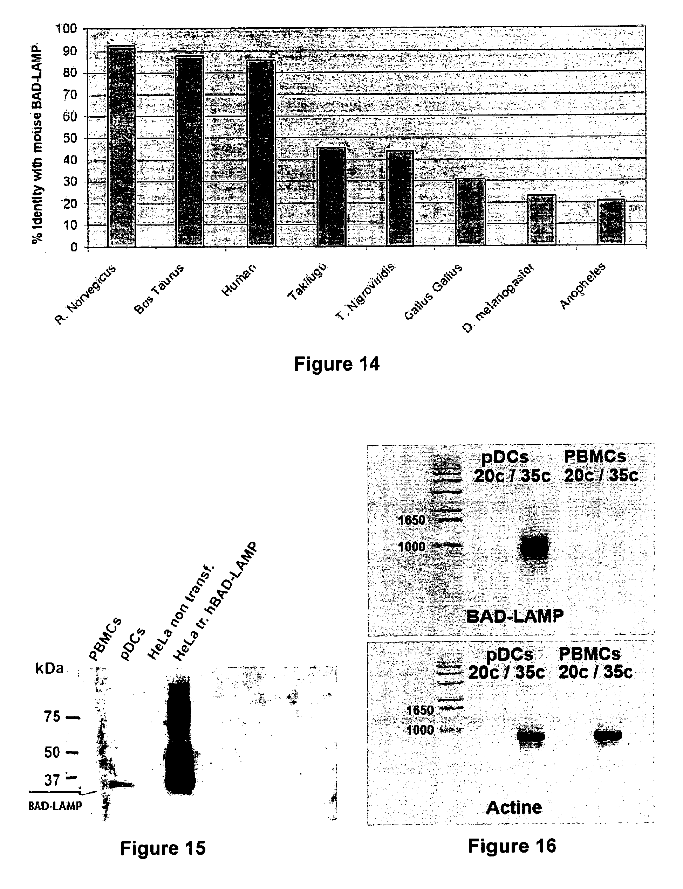

The percentage of identity of SEQ ID NO: 2, 6, 8, 16, 18, 20, 22, and 24 with respect to SEQ ID NO: 4 is represented in Figure 14.

In another embodiment, the above-defined isolated protein is represented by SEQ ID NO: 25 (a mutant BAD-LAMP protein referenced Tyr276Ala in the following

### Examples).

The present invention also relates to a fragment of an isolated protein consisting of:
- a sequence represented by SEQ ID NO: 2, or
- a homologous sequence which presents at least 20% identity to SEQ ID NO: 2, preferably at least 40%, more preferably at least 60%, most preferably at least 80%, provided that the homologous sequence represents a protein having an activity similar to that of *Homo sapiens* BAD-LAMP,
wherein said fragment comprises at least 10 amino acids.

In an embodiment, the invention relates to a fragment as defined above of an isolated protein represented by a sequence selected from the group consisting of SEQ ID NO: 2, SEQ ID NO: 4, SEQ ID NO: 6, SEQ ID NO: 8, SEQ ID NO: 10, SEQ ID NO: 12, SEQ ID NO: 14, SEQ ID NO: 16, SEQ ID NO: 18, SEQ ID NO: 20, SEQ ID NO: 22 and SEQ ID NO: 24.

In a preferred embodiment, the invention relates to a fragment as defined above of an isolated protein represented by a sequence selected from the group consisting of SEQ ID NO: 2, SEQ ID NO: 4, SEQ ID NO: 6, SEQ ID NO: 8 and SEQ ID NO: 10.

In another preferred embodiment, the invention relates to a fragment as defined above of an isolated protein represented by SEQ ID NO: 2 or SEQ ID NO: 4.

In another preferred embodiment, the above defined fragment is selected from the group consisting of KMTANQVQIPRDRSQC (SEQ ID NO: 26), KQIPRDRSQYKHMC (SEQ ID NO: 27), KMTANQVQIPRDRSQYKHMC (SEQ ID NO: 28), and SEQ ID NO: 31.

The present invention also relates to an isolated nucleic acid comprising or consisting of:
- a nucleotide sequence encoding an isolated protein or a fragment as defined above, or
- a homologous nucleotide sequence which presents at least 80% homology to said nucleotide sequence encoding an isolated protein as defined above, provided that said homologous nucleotide sequence encodes a protein presenting an activity similar to that of *Homo sapiens* BAD-LAMP; or
- a mutant nucleotide sequence which derives from said nucleotide sequence encoding an isolated protein as defined above by insertion, deletion or substitution of at least one nucleotide, provided that said mutant nucleotide sequence encodes a protein presenting an activity similar to that of *Homo sapiens* BAD-LAMP; or
- a hybridizing nucleotide sequence which hybridizes under stringent conditions with said nucleotide sequence encoding an isolated protein as defined above, provided that the complementary sequence of said hybridizing nucleotide sequence encodes a protein presenting an activity similar to that of *Homo sapiens* BAD-LAMP; or
- the complementary sequences of said isolated protein-encoding nucleotide sequence, homologous nucleotide sequence, mutant nucleotide sequence and hybridizing nucleotide sequence.

In an embodiment, the above-defined isolated nucleic acid is represented by a sequence selected from the group consisting of SEQ ID NO: 1 (*Homo sapiens* BAD-LAMP), SEQ ID NO: 3 (*Mus musculus* BAD-LAMP), SEQ ID NO: 5 (*Rattus norvegicus* BAD-LAMP), SEQ ID NO: 7 (*Bos taurus* BAD-LAMP), SEQ ID NO: 9 (*Pan troglodytes* BAD-LAMP), SEQ ID NO: 11 (*Monodelphis domestica* BAD-LAMP), SEQ ID NO: 13 (*Xenopus tropicalis* BAD-LAMP), SEQ ID NO: 15 (*Gallus gallus* BAD-LAMP), SEQ ID NO: 17 (*Anopheles gambiae* BAD-LAMP), SEQ ID NO: 19 (*Tetraodon viridis* BAD-LAMP), SEQ ID NO: 21 (*Takifugu rubripes* BAD-LAMP) and SEQ ID NO: 23 (*Drosophila melanogaster* BAD-LAMP).

In a preferred embodiment, the above-defined isolated nucleic acid is represented by a sequence selected from the group consisting of SEQ ID NO: 1, SEQ ID NO: 3, SEQ ID NO: 5, SEQ ID NO: 7, and SEQ ID NO: 9.

In another preferred embodiment the above-defined isolated nucleic acid is represented by SEQ ID NO: 1 or SEQ ID NO: 3.

The present invention also relates to a ligand comprising at least one binding moiety, wherein said at least one binding moiety is specific for an isolated protein as defined above, or for a protein encoded by an isolated nucleic acid as defined above.

In an embodiment, the above-defined ligand further comprises at least one detectable marker.

In another embodiment, the above-defined ligand consists of at least one binding moiety linked to at least one detectable marker.

In a further embodiment, the above-defined ligand consists of at least one binding moiety.

The present invention also relates to the use of a ligand as defined above for detecting human plasmacytoid dendritic cells in a biological sample and/or for purifying human plasmacytoid dendritic cells from a biological sample.

The present invention also relates to the use of an isolated nucleic acid as defined above, which is liable to bind under stringent conditions to a RNA encoding a protein represented by SEQ ID NO: 2, for detecting human plasmacytoid dendritic cells in a biological sample.

The present invention also relates to a kit for the detection and/or the purification of human plasmacytoid dendritic cells comprising:
- a ligand as defined above,
- a control consisting of an isolated protein as defined above, or a fragment as defined above,
- at least one suitable buffer.

The present invention also relates to a method for detecting human plasmacytoid dendritic cells in a biological sample, comprising a step of detecting the expression of an isolated protein as defined above in cells of the biological sample, whereby the cells in which the expression of a protein as defined above is detected are human plasmacytoid dendritic cells.

In an embodiment, the above-defined method for detecting human plasmacytoid dendritic cells in a biological sample, comprises the following steps:
- contacting the biological sample with at least one ligand as defined above to form a mixture,
- incubating the mixture in conditions allowing the formation of complexes between the ligand and an isolated protein as defined above,
- detecting the complexes formed between the ligand and the protein,
   whereby the presence of human plasmacytoid dendritic cells is detected.

The present invention also relates to a method for purifying human plasmacytoid dendritic cells from a biological sample, comprising the following steps:
- detecting the expression of an isolated protein as defined above in cells of the biological sample,
- selecting cells in which the expression the protein is detected,
   whereby human plasmacytoid dendritic cells are purified.

In an embodiment, the above-defined method for purifying human plasmacytoid dendritic cells comprises the following steps:
- contacting a biological sample with at least one ligand as defined above to form a mixture,
- incubating the mixture in conditions allowing the formation of complexes between the ligand and a protein as defined as defined above,
- separating the complexes between the ligand and the protein from the rest of the mixture,
   whereby human plasmacytoid dendritic cells are purified.

The present invention also relates to a method, in particular an *in vitro* method, for diagnosing the presence of a tumor in an individual, comprising a step of detecting the expression of an isolated protein as defined above in cells of a tissue of the individual suspected of harbouring a tumor, wherein the expression of the isolated protein is indicative of the presence of a tumor in said tissue.

The present invention also relates to a method, in particular an *in vitro* method, for prognosing the evolution of a tumor or for evaluating the risk of metastasis of a tumor in an individual, comprising a step of detecting the expression of an isolated protein as defined above in cells of a tissue harbouring the tumor, wherein the expression of the isolated protein is indicative of a growth of the tumor or of an increased risk of metastasis of the tumor, as compared to tumors which are not included in tissues which cells express an isolated protein as defined above.

In an embodiment of the above-defined methods for diagnosing the presence of a tumor, for prognosing the evolution of a tumor, or for evaluating the risk of metastasis of a tumor, the detection of the expression of the isolated protein is carried out on a biological sample taken from the individual.

### Description of the figures

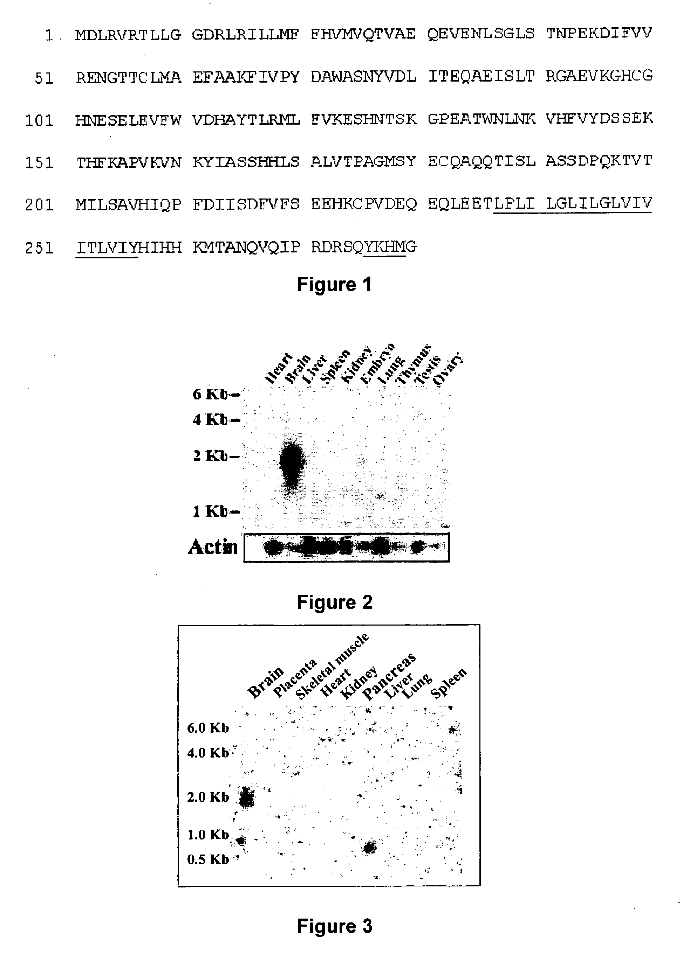

Figure 1 represents the peptide sequence of mouse BAD-LAMP (from the N-terminus to the C-terminus). The first underlined part of the sequence (starting from the N-terminus) represents the transmembrane domain and the second underlined part of the sequence represents a consensus internalization sequence and endosomal targeting signal.
Figure 2 represents a northern blot of various mouse tissue samples (on top) using a radio-labelled BAD-LAMP mRNA-specific probe. A spot corresponding to BAD-LAMP mRNA is represented by an arrow.
Figure 3 represents a northern blot of various human tissue samples (on top) using a radio-labelled BAD-LAMP mRNA-specific probe. A spot corresponding to BAD-LAMP mRNA is represented by an arrow.

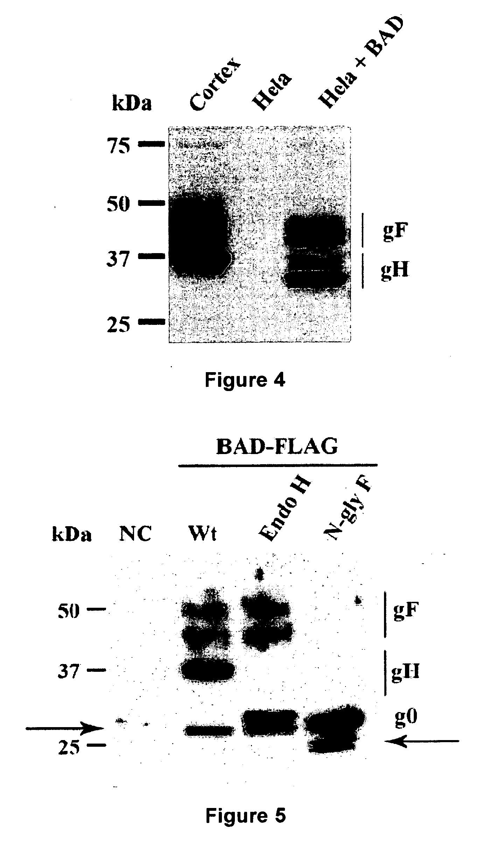

Figure 4 represents an immunoblot performed with a polyclonal antibody raised against a predicted peptide of the BAD-LAMP cytoplasmic tail. Lysates of mouse adult cortex and BAD-LAMP transfected HeLa cells display several bands migrating above 35 kDa, while no reactivity is observed in control untransfected cells, the lowest form accumulating in the transfected cells is probably due to ER accumulation.
Figure 5 represents an immunoblot performed with a polyclonal antibody raised against a predicted peptide of the BAD-LAMP cytoplasmic tail. HeLa cells transfected with FLAG-BAD-LAMP cDNA are lysed and immunoprecipitated with anti-FLAG antibody. Immunoprecipitated material is treated with endoglycosidase H (endo H) or N-glycosidase F (N-gly F) prior immunoblotting with anti-BAD-LAMP. In untransfected cells (NC), just the anti-FLAG IgG band (arrow) is observed, while several isoforms of BAD-LAMP are immunoprecipitated in transfected cells (Wt). Endo H treatment shows that the major band is endo H sensitive (gH) thus probably accumulating in the ER. The higher molecular weight bands are all N-gly F sensitive (gF). All BAD-LAMP isoforms are glycosylated and its native molecular weight is as predicted close to 32kDa (g0). The anti-FLAG IgG band are also N-gly F sensitive, arrows.

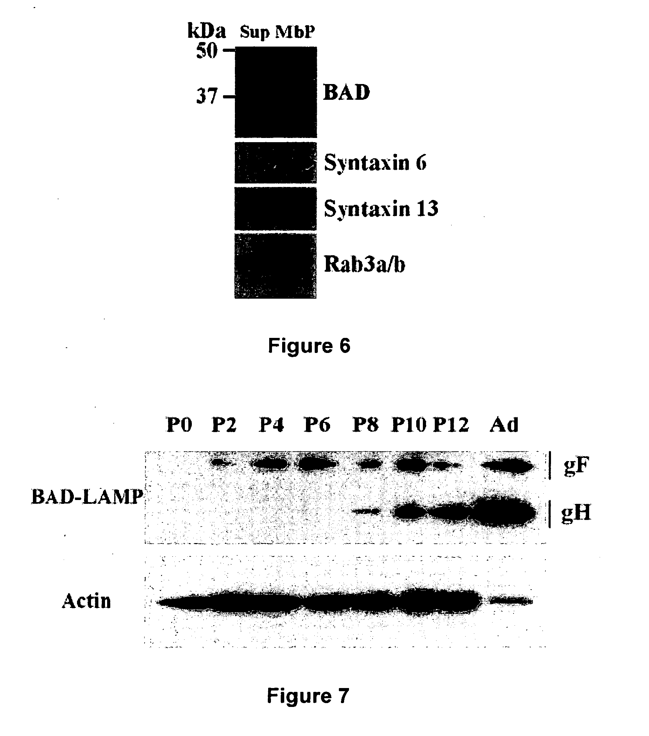

Figure 6 represents an immunoblot performed with a polyclonal antibody raised against a predicted peptide of the BAD-LAMP cytoplasmic tail. After fractionation and isolation of cortical membranes, BAD-LAMP is found present exclusively in the membrane pellet (MbP) and not in the supernatant (Sup). Control syntaxin 6 and syntaxin 13 were also found in the membrane pellet, while Rab3 was distributed in the two fractions due to its shuttling nature.
Figure 7 represents an immunoblot performed with a polyclonal antibody raised against a predicted peptide of the BAD-LAMP cytoplasmic tail. Post-natal mouse cortex extracts were immunoblotted with BAD-LAMP polyclonal antibody. BAD-LAMP expression is increased from birth to adulthood (P0 to Ad).

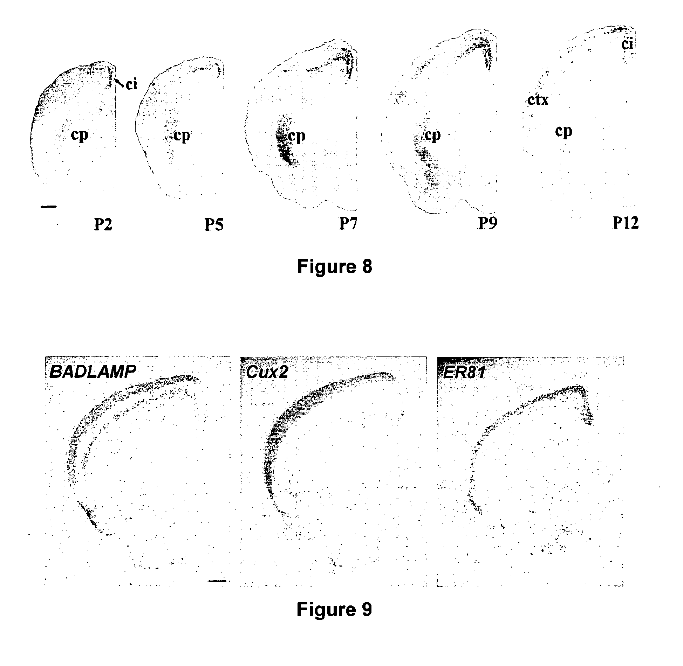

Figure 8 represents *in situ* hybridization for BAD-LAMP on coronal post-natal brain sections from P2 to P12. Hemisections are presented. Bad-lamp expression signal appears at P2 in the cingulate cortex (ci) and extended ventrally during the first post-natal weeks as a superficial and a deep band in the cortex (ctx). In subcortical structure, BAD-LAMP is expressed transiently in the caudate putamen (cp). Bar: 500 µm.
Figure 9 represents *in situ* hybridization for BAD-LAMP (top panel), Cux2 (middle) and ER81 (bottom) on adult brain coronal hemisections.

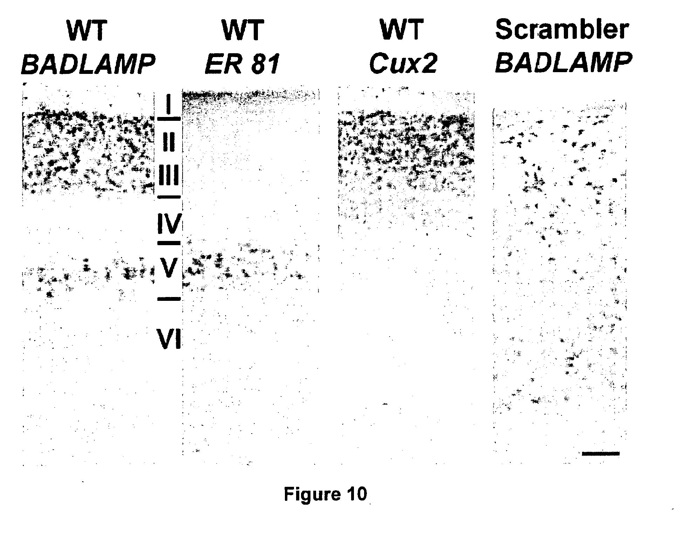

Figure 10 represents high magnification views of *in situ* hybridization on wild type cortex shown in Figure 9 and *in situ* hybridization for BAD-LAMP in Scrambler cortex. BAD-LAMP is expressed in layers II, II and V, but excluded from layers IV and VI. In the Scrambler cortex, the entire cortex appears disorganized. However, the typical inversion of cortical layers is reflected by the altered BAD-LAMP staining, demonstrating that projection neurons express the protein.

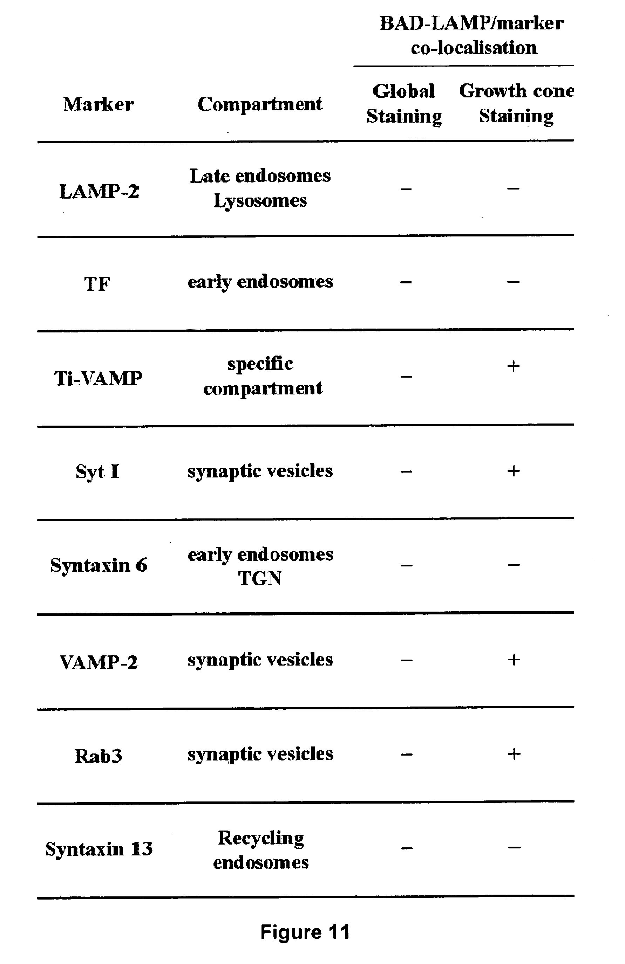

Figure 11 represents a list of the different molecules imaged in cortical neurones by confocal microscopy is given along with their level of co-localization with BAD-LAMP.

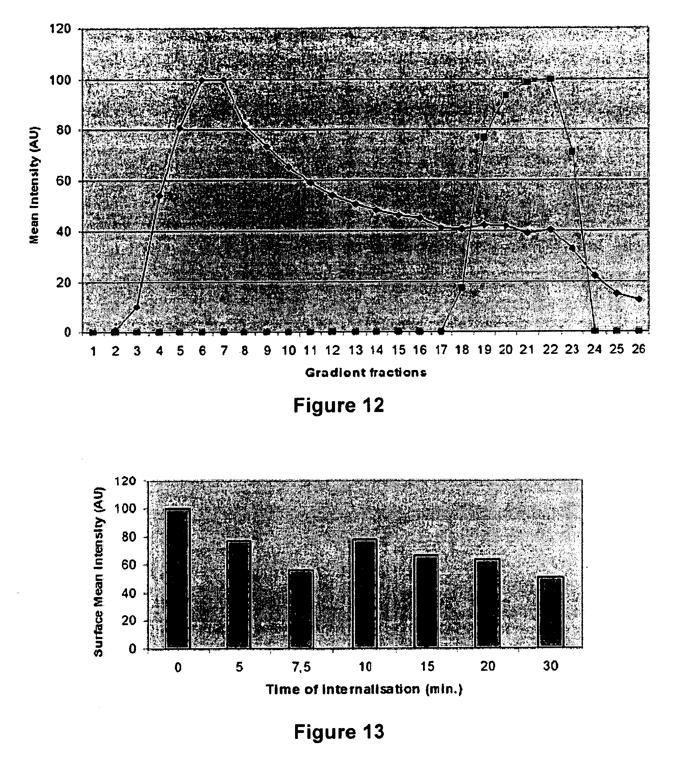

Figure 12 represents a subcellular Percoll gradient fractionation showing that in transfected HeLa cells BAD-LAMP is mostly localized in light density fractions and is absent from heavy density fractions containing the lysosomal enzyme β-hexosaminidase.
Figure 13 shows a representative FACS staining experiment (of 3), showing the level of internalised FLAG antibody against time at the surface of FLAG-BAD-LAMP transfected HeLa cells. The timing of internalization and the recovery of surface staining at 10 min indicates the BAD-LAMP recycles in HeLa cells.
Figure 14 represents the percentage of amino acid identity of *Rattus norvegicus* (SEQ ID. NO: 6), *Bos Taurus* (SEQ ID NO: 8), *Homo sapiens* (SEQ ID NO: 2), *Takifugu rubripes* (SEQ ID NO: 9), *Tetraodon nigroviridis* (SEQ ID NO: 10), *Gallus galius* (SEQ ID NO: 11), *Drosophila melanogaster* (SEQ ID NO: 12), and *Anopheles gambiae* (SEQ ID NO: 13) BAD-LAMPs with respect to *Mus musculus* (SEQ ID NO: 4) BAD-LAMP.
Figure 15 represents an immunoblot of human peripheral blood monocyte, human plasmacytoid DCs, HeLa cells and HeLa cells transfected with BAD-LAMP cDNA extracts using a monoclonal anti-BAD-LAMP antibody.
Figure 16 represents the results of RT-PCR experiments (20 or 35 cycles) performed with BAD-LAMP specific primers (upper panel) or actine specific primers (lower panel) on peripheral blood monocyte (PBMC) and human plasmacytoid DC (pDC) extracts.

### Detailed description of the invention

As intended herein "a protein having an activity similar to that of *Homo sapiens* BAD-LAMP" relates to a protein which is liable to perform at least one of the tasks the *Homo sapiens* BAD-LAMP protein is liable to perform naturally. Generally, evidencing that a protein has an activity similar to that of BAD-LAMP can be established if said protein, upon its expression, can restore at least partially a normal phenotype in a cell which phenotype has been impaired by suppressing or diminishing the expression of *Homo sapiens* BAD-LAMP in said cell. Methods for suppressing or diminishing the expression of *Homo sapiens* BAD-LAMP in a cell and for expressing a protein in a cell are well-known to the man skilled in the art.

Preferably, the protein having an activity similar to that of *Homo sapiens* BAD-LAMP is such that it presents the same tissular or cellular localization than that of *Homo sapiens* BAD-LAMP. Determining the tissular or cellular localization of the protein having an activity similar to that of *Homo sapiens* BAD-LAMP can be performed by numerous methods well-known to the man skilled in the art, such as immunocytochemistry methods using specific antibodies to said protein having an activity similar to that of *Homo sapiens* BAD-LAMP. Immunochemistry methods applied to the determination of the tissular or cellular localization of *Homo sapiens* BAD-LAMP are notably described in the following Examples. Preferably, if naturally found in a living organism, the protein having an activity similar to that of *Homo sapiens* BAD-LAMP is expressed in the central nervous system, such as the brain, or in plasmacytoid dendritic cells, of said organism. Preferably also, the protein having an activity similar to that of *Homo sapiens* BAD-LAMP is associated to plasma and/or vesicular membranes of the cells in which it is expressed.

As intended herein a first peptide sequence which presents at least x % identity to a second peptide sequence means that x % represents the number of amino acids of the first sequence which are identical to their paired amino acids of the second sequence when the two sequences are optimally aligned pairwise, with respect to the total length of the second peptide sequence. As intended herein two sequences are said to be optimally aligned pairwise when x is maximal.

As intended herein a first nucleic sequence which presents at least y % homology to a second nucleic acid sequence means that x % represents the number of nucleotides of the first sequence which are identical to their paired nucleotides of the second sequence when the two sequences are optimally aligned pairwise, with respect to the total length of the second nucleic acid sequence. As intended herein two sequences are said to be optimally aligned pairwise when y is maximal.

Alignment can be performed manually or automatically. Algorithms for automated aligning of peptide or nucleic acid sequences are well known to the man skilled in the art and are for example described in Altschul et al. (1997) Nucleic Acids Res. 25:3389 and implemented by softwares such a the Blast software.

Stringent conditions for nucleic acid hybridization are well known to the man skilled in the art and are notably described in Sambrook & Rusell (2001) "Molecular Cloning: A Laboratory Manual", 3rd edition, Cold Spring Harbor Laboratory Press. By way of example, suitable stringent conditions according to the invention, with respect to a given nucleic acid, consist in a hybridization carried out at temperatures 20°C below the calculated melting temperature (Tm) for said nucleic acid in the same medium than that used for calculating the Tm.

As intended herein, an isolated protein according to the invention can also comprise detectable markers such as Green Fluorescent Protein (GFP) or epitopic moieties such as FLAG® tags.

SEQ ID NO: 25 represents a mutant *Mus musculus* BAD-LAMP comprising a Tyr to Ala substitution at position 276, which activity is similar to that of BAD-LAMP as intended herein.

Fragments according to the invention are useful in the frame of the generation of specific ligands, such as antibodies, to isolated proteins as defined above.

As intended herein a "ligand" refers to any compound liable to specifically bind to an isolated protein as defined above. The part or parts of the ligand which effectively bind to the isolated protein as defined above are named "binding moiety" or "binding moieties". A ligand according to the invention can thus comprise or be consisting of one or several binding moieties. In particular, when a ligand according to the invention comprises one or several binding moieties, it can also comprises at least one "detectable marker", that is a moiety the presence of which can be readily evidenced according to methods well known to the man skilled in the art.

Preferably, in the ligands according to the invention, the at least one binding moiety is specific for a protein represented by a sequence selected from the group consisting of SEQ ID NO: 2, SEQ ID NO: 4, SEQ ID NO: 6, SEQ ID NO: 8, and SEQ ID NO: 10.

Also preferably, the at least one binding moiety is selected from the group consisting of an antibody, an antigen-specific antibody fragment, a scFv, and an aptamer.

As intended herein an "antigen-specific antibody fragment" relates to an antibody fragment which retains its specific binding properties towards an isolated protein according to the invention. Such fragments notably encompass Fab fragments (which can be produced by papain cleavage of antibodies), F(ab')₂ fragments (which can be produced by pepsin cleavage of antibodies) or Fab' fragments (which can be produced by pepsin cleavage of antibodies followed by a reducing treatment).

A "scFv" relates to a single-chain variable fragment of an antibody, that is an immunoglobulin short chain variable region and an immunoglobulin large chain variable region linked together by a peptide.

An "aptamer" relates to a nucleic acid molecule, in particular a ribonucleic acid molecule.

Methods for producing an antibody, an antigen-specific antibody fragment, a scFv, or an aptamer are well-known to the man skilled in the art.

More preferably the at least one binding moiety is a monoclonal antibody.

General methods for producing monoclonal antibodies are well known to the man skilled in the art and are notably described by Kohler & Milstein (1975) *Nature* 256:495 or illustrated in the following Examples.

Most preferably the monoclonal antibody is produced by one of the following hybridomas:
- a hybridoma deposited at the Collection Nationale de Cultures de Microorganismes (CNCM, Paris, France) under the Budapest Treaty on October 19, 2006 with accession number I-3679, or
- a hybridoma deposited at the Collection Nationale de Cultures de Microorganismes (CNCM, Paris, France) under the Budapest Treaty on October 19, 2006 with accession number I-3684.

Preferably, the above-defined at least one detectable marker is selected from the group consisting of an enzyme, a chromophore, and a fluorophore.

As intended herein the expression "detecting plasmacytoid dendritic cells" relates to the unambiguous identification of plasmacytoid dendritic cells in a sample with respect to other cell types which might be comprised in said sample

As intended herein the expression "purifying plasmacytoid dendritic cells" relates to the separation of plasmacytoid dendritic cells comprised in a sample from other cells which might also be present in said sample.

Methods for diagnosing the presence of a tumor, for prognosing the evolution of a tumor, or for evaluating the risk of metastasis of a tumor according to the invention, take advantage of the specific expression of *Homo sapiens* BAD-LAMP by plasmacytoid dendritic cells, which plasmacytoid dendritic cells have been found to be associated to various tumors such as breast tumors (Bell et al. (1999) J. Exp. Med. 190:1417) and melanomas (Vermi et al. (2003) J. Pathol. 200:255). Furthermore, infiltration of breast tumor by plasmacytoid dendritic cells has been shown to correlate with growth and dissemination (metastasis) of the tumor, and to overall correlate with an adverse outcome for the patient which harbours the tumor (Treilleux et al. (2004) Clin . Cancer Res. 10:7466).

As intended herein, "biological samples" in which the presence of plasmacytoid dendritic cells is sought preferably do not originate from brain tissues.

### EXAMPLES

### Example 1

### Materials and methods

### Bioinformatics

BAD-LAMP protein sequence ID in Ensembl database : ENSMUSP00000061180. All LAMPs sequences were aligned using CLUSTALW package (EBI) and results were treated with TreeView for phylogeny. Image analysis was performed with the Image J software and the plugin JacoP designed by Fabrice Cordelières, Institut Curie, Orsay (France).

### Animals and Tissues

All animals were treated according to protocols approved by the French Ethical Committee.

CD1 mice (Iffa-Credo, France) were used to determine BAD-LAMP expression pattern. Disabled-1 deficient Scrambler mice have been purchased from Jackson Laboratories.

The day of the vaginal plug appearance was considered as E0.5 and the day of the birth is termed postnatal (P) 0.

For in situ hybridisation and immunohistochemistry, postnatal and adult brains were collected after the animals were anaesthetized with a lethal dose of Rompun/Imalgen 500 and intracardiacally perfused with 4% paraformaldehyde (PFA). Brains were further fixed in 4% PFA overnight. Adult brains were sectioned at 80 µm on a vibratome while P2 to P12 brains were cryoprotected in 20% sucrose/PBS, frozen in OCT compound and sectioned at 16 µm on a cryostat. Sections collected on Superfrost slides were treated as described below.

### Molecular biology

Northern blot was done with FirstChoice Northern Blot Mouse Blot I (Ambion) using a probe corresponding to exons 4, 5 and 6 of BAD-LAMP (clone IMAGE 2588577). 2 µg of Trizol extracted total mouse cortex RNA was used for reverse transcription with oligo(dT) primers. The cDNAs coding for BAD-LAMP were amplified after 30 cycles of PCR using Taq polymerase. Sense primer was ACC GGC CAC TTT GAG GGA (SEQ ID NO: 29) and antisense GGG GCG GCC TTT GCA GCA (SEQ ID NO: 30) (1,5 kbp). PCR products were cloned into pGEM-Teasy plasmid (Promega).

BAD-LAMP-GFP fusion construct was done in pEGFP-NI vector (Clontech). FLAG-BAD-LAMP was constructed using pTEJ-8-HAFLAG plasmid (Didier Marguet, Marseille).

Tyrosine mutant of BAD, FLAG-BAD-Tyr-276-Ala was realized by targeted PCR mutagenesis.

FLAG-BAD-LAMP cDNAs were transferred into pCX-MCS2 plasmid, a pCAAGS derived plasmid with an extended cloning site (a kind gift of Xavier Morin, Marseille). Dynamin-GFP wt plasmid and dynamin-GFP A44K were kindly given by M. McNiven, Rochester. RNAi constructs pSUPER AP-2 µ2 and pSUPER control were a gift from Dr Philippe Benaroch, Paris.

### In situ hybridization and immunohistochemistry

IMAGE clone 2588577 was used to make an antisense RNA probe. Antisense RNA probe for BADLAMP, Cux2 (Zimmer et al., 2004) and ER81 (Lin et al., 1998) were generated using the Dig-RNA labelling kit (Roche).

Single *in situ* hybridization and combined *in situ* hybridization with immunohistochemistry were described previously (Tiveron et al., 1996) (Zimmer et al., 2004) for all probes and the NeuN monoclonal mouse IgG (MAB377, Chemicon).

### Antibodies and immunocytochemistry.

A polyclonal rabbit anti-BAD-LAMP was raised in rabbit against two peptides of BADLAMP cytoplasmic tail, KMTANQVQIPRDRSQC (SEQ ID NO: 26) and KQIPRDRSQYKHMC (SEQ ID NO: 27).

Antisynaptotagmin I and anti-Rab3a/b antibodies was obtained from Dr P. Di Camilli, New Haven, anti-FLAG M2 antibody and anti-®-tubulin-Cy3 and FITC-cholera toxin B subunit (GM-1 staining) were obtained from Sigma, anti-VAMP2 was from SYSY, antisyntaxin 6 was from BD Transduction Laboratories, anti-PrP (6H4) was from Prionics, anti-syntaxin 13 was from Stressgen, anti-Thy1 was from Michel Pierres, Marseille, human Alexa 568-Tf was from molecular probes, mouse FITC-Tf was from Rockland, anti-NCAM H28 from Dr. C. Goridis, Paris, Anti-Ti-VAMP from Dr. T. Galli, Paris, Rat anti mouse LAMP2 from Dr I. Mellman, New Haven and anti human LAMP-1 from Abcam. All FITC and Cy3-5 secondary antibodies were from Jackson lmmunoresearch. All Alexa secondary antibodies were from Molecular Probes (USA).

Immunofluorescence and confocal microscopy was performed with a Zeiss LSM 510 as described (Cappello et al., 2004). Vibratome adult brain sections were immunostained with rabbit anti-BAD-LAMP and mouse anti-MAP2.

### Cell culture

HeLa cells were grown in DMEM containing 10% FCS.

Cortical neurons were prepared from E15.5 embryonic cortices. Cortices were dissected out in HBSS, treated for 15 min at 37°C in Trypsin/EDTA-HBSS (Invitrogen), washed once in NeuroBasal medium (NB, Invitrogen) complemented with 10 % Horse Serum to block trypsin activity, washed once more in NB alone. Cortical neurons were dissociated, plated on glass coverslips in NB complemented with B27 complement, 2 mM LGlutamine and 50µg/ml Pencillin/Streptamycin (InVitrogen) and cultured for 3 days at 37°C, 5% CO2. Coverslips were coated overnight with poly-L-Lysine (10 µg/ml).

### Transfection and internalisation experiments

Neurons were electroporated using Amaxa Nucleofactor Kit according to manufacturer's instructions. Hela cells were grown on coverslips and transfected using lipofectamine 2000 (Invitrogen) using manufacturer's protocol. After 8 to 24 hours of transfection cells were processed to study internalisation kinetics or fixed using paraformaldehyde 3%.

Internalisation assays were performed using FITC conjugated transferrin or unconjugated antibodies. The cells were first incubated for 20 min at 37°C in DMEM/100mM Hepes to eliminate endogenous transferrin. Cells were incubated 15 min at 4°C with ligand and/or antibody and washed twice in icecold PBS before incubation with DMEM, 1% BSA, 100mM Hepes at 37°C during different times prior fixation and immunocytochemistry. Neurons were processed identically in NB medium.

Cortical neuron biotinylation was performed using EZ-Link Sulfo-NHS-Biotin kit (PIERCE) with 15 min reaction at 4°C followed by 3 washes with ice-cold PBS containing 10 mM Glycine. Cells were incubated for 5 and 45 min at 37°C to allow endocytosis of biotinylated membrane proteins, prior fixation and immunostaining.

### Immunoblots and immunoprecipitation

1% Triton X-100 cell extracts complemented with protease inhibitors cocktail (Roche) were immunoblotted after separation by 12% or 7-17% gradient SDS-PAGE.

Immunoprecipitation with anti-flag antibody and N-glycosidase F or endoglycosidase H (Calbiochem) treatment were performed as described (Cappello et al., 2004).

### Results

### Sequence identification

During a bioinformatics homology search to identify Lysosomal-associated molecules (LAMP), several mouse ESTs coding for overlapping sequences were isolated. After reconstitution of the full length sequence and extensive analysis, the Inventors identified a potential open reading frame (ORF) coding for a new member of the LAMP family (SEQ ID NO: 3). The putative ORF codes for a protein of 280 aa (SEQ ID NO: 4) and is predicted to contain a transmembrane domain (aa 237-256) and a cytoplasmic tail of 24 residues (aa 257-280) **(****Figure 1****).** This predicted cytoplasmic domain contains a YKHM motif (aa 276-279) corresponding to a classical YXXO internalization sequence and endosomal targeting signal **(****Figure 1****)** The sequence contains 4 highly conserved cysteine residues separated by a fixed number of amino acids which are likely to form the characteristic internal di-sulfide bonds required to form LAMP-like domains.

One of the hallmarks of LAMP proteins is their important level of sugar modification. In agreement, the protein was also predicted to contain 3 consensus N-glycosylation sites. The nucleotide sequence was found to be homologous at the level of 41.6% and 46.4% with LAMP 1 and LAMP 2 respectively, the founding members of the family, while protein alignments displayed 24.7% and 21.2% of identity. Based on its size and the presence of one LAMP domain, the protein was placed on an evolutionary classification tree between LAMP1 and DC-Hil sequences, clearly identifying it as a new member of the LAMP family. DC-Hil (which shares 15.5% identity with LAMP1), is a dendritic cell-specific molecule functioning as an integrin ligand.

Analysis of the expression of the identified mouse mRNA by northern blot using various mouse tissues indicated that its presence mainly, if not exclusively, in the adult brain **(****Figure 2****).** Expression in the embryo was extremely low. The detected mRNA corresponds to a unique transcript of around 1.8kb with no apparent alternative spliced forms.

The following homologues have been identified:
SEQ ID NO: 2 (Homo sapiens BAD-LAMP), SEQ ID NO: 6 (*Rattus norvegicus* BAD-LAMP), SEQ ID NO: 8 *(Bos taurus* BAD-LAMP), SEQ ID NO: 10 *(Pan troglodytes* BAD-LAMP), SEQ ID NO: 12 (*Monodelphis domestica* BAD-LAMP), SEQ ID NO: 14 (*Xenopus tropicalis* BAD-LAMP), SEQ ID NO: 16 (*Gallus gallus* BAD-LAMP), SEQ ID NO: 18 (*Anopheles gambiae* BAD-LAMP), SEQ ID NO: 20 (*Tetraodon viridis* BAD-LAMP), SEQ ID NO: 22 (*Takifugu rubripes* BAD-LAMP) and SEQ ID NO: 24 (*Drosophila melanogaster* BAD-LAMP).

Analysis of the expression of the human mRNA by northern blot is presented in **Figure 3**. As in mice, the expression of the mRNA appears to be essentially localized in the brain. **However, some expression is also seen in the pancreas.**

Based on its relationship to the LAMP family and its restricted pattern of expression, the above-identified protein was named BAD-LAMP, for Brain-AssociateD LAMP.

### BAD-LAMP is a glycosylated membrane associated protein

To investigate further BAD-LAMP distribution and function, antibodies were raised against peptide epitopes present in its cytoplasmic tail. These antibodies were characterized by immunoblot of HeLa cells transfected with the cDNA coding for mouse BAD-LAMP (**Figure 4**). Several bands were detected in extracts from transfected cells, while control extracts remained non-reactive.

Brain extracts displayed also several bands, mostly corresponding to those observed in transfected cells, confirming the existence of several isoforms of BAD-LAMP.

The detected proteins had a significantly higher molecular weight than the one predicted from the primary sequence of BAD-LAMP (31.7 kDa). In order to define the nature of these post-translational modifications and in absence of immuno-precipitating antibodies, an N-terminally tagged form of BAD-LAMP (FLAG-BAD-LAMP) was transfected allowing efficient immuno-precipitation and treatment with endoglycosidase H (Endo H) and N-glycosidase F (N-gly F).

Immuno-precipitated FLAG-BAD-LAMP was shown to be heavily glycosylated **(****Figure 5****).** The major form of the protein (38 kDa, gH) remained Endo H sensitive, thus reflecting endoplasmic reticulum (ER) retention due to over-expression. The two higher additional bands (47 and 53 kDa, gF) were Endo H resistant but remained N-gly F sensitive, as indicated by the accumulation of a fully trimmed 31 kDa protein (g0) after treatment. Transfected BAD-LAMP is therefore heavily glycosylated on at least 2 of its 3 acceptor sites, a situation likely to be shared with endogenous BAD-LAMP detected in brain.

Although glycosylation was in support of BAD-LAMP membrane association, the membrane bound nature of BAD-LAMP was demonstrated by submitting mouse cortex post-nuclear supernatants to high speed ultracentrifugation in which BAD-LAMP was found associated with the membranes pellets like other membrane associated molecules such as Rab 3a, syntaxin 6 and syntaxin 13 **(****Figure 6****).**

Thus BAD-LAMP, is a glycosylated LAMP-like molecule associated to cortical membranes.

### BAD-LAMP is expressed in neurons of specific cortical layers after birth.

Analysis of mouse brain extracts by immunobloting revealed that levels of BAD-LAMP increased strongly after birth (P0) reaching its maximum level at adulthood, but being already strongly expressed at P10-P12 stages **(****Figure 7****).**

*In situ* hybridization was used to investigate in detail the expression pattern of BAD-LAMP in the developing mouse forebrain. First expression of BAD-LAMP was found at P2 in the cingulate cortex, where a thin band of cells in an intermediate position is labelled **(****Figure 8****).** At P5, expression extended ventrally into the cortical plate. Furthermore, the caudate putamen showed a punctuate expression of BAD-LAMP transcripts. This expression pattern was maintained at P7, when an additional broad band of large and strongly BAD-LAMP-positive cells appeared in superficial parts of the cortical plate. Although the cortical expression intensified until P9, no major regional changes in BAD-LAMP expression were obvious during this period. At P12 expression of BAD-LAMP in the striatum ceased, while expression further intensified in the cortex. This expression pattern was stable until adulthood.

Altogether, this expression pattern is in agreement with a function of BAD-LAMP not in early steps of brain development, like neurogenesis and cell migration, but potentially during terminal steps of neuronal differentiation and neuronal function.

Within the adult cortex, the homogenous staining in outer regions of the cortical plate, as well as in a more restricted band of cells localized centrally was suggestive of an expression in neurons of specific cortical layers. Well-known markers for cortical layers were used to further characterize the respective populations. Comparison of the expression of BAD-LAMP to that of CUX2, a marker for layers II-IV, showed that the BAD-LAMP domain is included in the CUX2 domain and confined to its outer part **(****Figures 9** **and** **10****).** Thus, BAD-LAMP is expressed in the upper layers II and III of the neocortex, but is excluded from layer IV.

Furthermore, comparison to the layer V marker ER81 **(****Figures 9** **and** **10****)** showed a perfect overlap, demonstrating that the deeply positioned BAD-LAMP-positive population is located in layer V.

The size of the BAD-LAMP-positive cells in the respective cortical layers was suggestive of neuronal cells. To confirm this observation the expression of BAD-LAMP was investigated in Scrambler mice. These animals show a well described inversion of the layers of cortical projection neurons, with upper layer neurons (layers II-IV) positioned deep while deep layer neurons (layers V and VI) are positioned superficially (Rice and Curran, 1999).

The organization of BAD-LAMP positive cells in the Scrambler cortex was strikingly altered (Figure 10). Small, lighter stained cell bodies were displaced towards the ventricular side, while larger and stronger labelled cells were merely found at the pial side of the cortex. This pattern was in agreement with an inversion of the position of BAD-LAMP-positive cells and suggestive of a projection neuron identity. Furthermore, all BAD-LAMP positive cells in the cortex coexpressed the neuronal marker NeuN, again confirming the neuronal identity of labelled cells.

BAD-LAMP protein distribution in cortical brain sections further confirmed its expression in specific layers. Higher magnification at the confocal level showed that protein was found at the plasma membrane and in vesicles mostly confined to the somatodendritic compartment as delineated by MAP2 staining. Within dendrites, BAD-LAMP was enriched in vesicles clustered in defined domains. BAD-LAMP mostly accumulated within the boundaries of specific neuronal areas *in vivo.*

### BAD-LAMP is distributed in specific domains of cortical neurons.

In order to confirm the relevance of the above observations, embryonic cortical neurons were explanted and BAD-LAMP sub-cellular distribution was investigated after 3 days of culture. Due the particular clustered distribution of BAD-LAMP, the distribution of proteins known to partition in different cellular domains such as lipid microdomain associated proteins (Madore et al., 1999) was also investigated.

Using confocal microscopy, it was found that semi-ordered lipid microdomain residents such as PrP, N-CAM, as well as the ganglioside GM-1 (stained with cholera toxin, CT) were enriched in zones excluding BAD-LAMP vesicles. This observation was particularly striking with CT staining and N-CAM, which accumulated almost exclusively in areas negative for BAD-LAMP.

In contrast, at this low magnification, Thy-1, a molecule representing ordered lipid microdomain-associated proteins, displayed an overlapping distribution with BAD-LAMP.

However, at higher magnification, no direct co-localization of Thy-1 and BAD-LAMP molecules could be observed. Instead, accumulation of BAD-LAMP-containing vesicles was revealed directly underneath Thy-1 enriched area at the plasma membrane.

BAD-LAMP containing vesicles therefore accumulate in cellular zones, defined by the presence of Thy-1 at the plasma membrane, while they are segregated away from the detergent resistant microdomains containing most of PrP, GM-1 and N-CAM (Madore et al., 1999).

BAD-LAMP distribution and microdomain organization seemed to be closely linked. Cholesterol depletion efficiently affects lipid microdomains and was therefore tested for its ability to influence BAD-LAMP distribution.

Cortical neurons were treated for 2h with cholesterol-oxydase prior to immunostaining and confocal microscopy visualization. As expected, cholesterol depletion had a potent effect on GM-1 distribution at the plasma membrane. Moreover BAD-LAMP vesicular staining was also deeply affected, displaying an extensive co-localization with GM-1, which was never observed in normal conditions. Thus, microdomain organization at the membrane and clustering of BAD-LAMP-positive vesicles appeared to be directly linked. However BAD-LAMP distribution is not dependent on the neuronal polarity.

### The cytoskeleton controls the distribution of BAD-LAMP vesicles

This particular organization was likely to be maintained with the active participation of the cytoskeleton and/or associated proteins. In order to test this hypothesis, several candidate molecules were followed by confocal microscopy in cortical neurons.

Surprisingly, BAD-LAMP containing-vesicles clustered within punctuate zones delimited by staining with the Smi31 antibody. Smi31 detects phosphorylated epitopes present in neurofilament H and in the microtubule-associated molecule 1 B (Mab1 B) (Fischer and Romano-Clarke, 1990). Although the precise function of MAP1B phosphorylation is still debated, experimental evidences suggest a role in regulating microtubules and actin dynamics as well as being necessary for axonal growth (Dehmelt and Halpain, 2004) (Del Rio et al., 2004). The perfect overlapping distribution of BAD-LAMP and Smi31 strongly suggests that microtubules or actin play an important role in the clustering of BAD-LAMP positive vesicles.

BAD-LAMP positive vesicles were found in close vicinity of the microtubule network, mirroring by their accumulation the intensity of the tubules. A treatment with the microtubule depolymerizing agent nocodazole was thus carried out. Nocodazole induced a strong redistribution of BAD-LAMP containing vesicles and a loss of BAD-LAMP staining intensity in cortical neurons. Thus the microtubule network influences the positioning of BAD-LAMP vesicles. Lipid microdomain organization and BAD-LAMP distribution in cortical neurons are therefore linked, use the microtubule network and possibly depend on MAP-1 B phosphorylation for their regulation.

### BAD-LAMP defines a specific subset of early endosomes.

The unusual distribution of BAD-LAMP vesicles led the inventors to investigate their relationship with other types of sub-cellular compartments. The inventors focused primarily on endocytic organelles, likely to be relevant to a transmembrane molecule bearing a YXXΦ motif in its cytoplasmic tail.

BAD-LAMP could not be detected in classical endosomal compartments (Figure 11), as judged from its lack of co-localization with LAMP-2 (late endosomes and lysosomes), internalized transferrin-FITC or syntaxin 13 (sorting and recycling endosomes (Hirling et al., 2000). BAD-LAMP was not found in more specialized endocytic compartment such as TI-VAMP positive vesicles (Coco et al., 1999). Co-labeling with synaptic vesicles proteins such as synaptotagmin I, Rab 3a, VAMP 2 revealed some level of co-localization with BAD-LAMP in the growth cones. Interestingly, co-localization was not observed in other cellular areas and a similar overlapping distribution in the growth cone was observed with TI-VAMP, which is not found enriched in synaptic vesicles (Coco et al., 1999). Thus, this co-distribution in the growth cone probably reflects the difficulty to segregate, at this optical resolution, individual carrier vesicles congregating in the same area of the cone, rather than a true co-localization in the same vesicles. Pre-and post-synaptic transport carriers are derived from trans-Golgi network (TGN) vesicles, which aggregate at initial contacts between axons and dendrites (Sytnyk et al., 2002).

The inventors therefore attempted to associate BAD-LAMP with other known vesicular markers of these pathways, such as syntaxin 6 or N-CAM (Sytnyk et al., 2004). Co-localization of BAD-LAMP with any of these markers **(****Figure 11****)** failed, suggesting that the molecule is sorted in an uncharacterized type of vesicles, which can accumulate in the growth cone of developing axon, as well as in defined and organized domains along the cellular processes.

BAD-LAMP distribution at the plasma membrane as well as in localized intracellular vesicles suggested a possible shuttling of the molecule between the cell surface and the vesicles. The co-localization of GM-1 and BAD-LAMP upon cholesterol depletion suggests that BAD-LAMP vesicles are accessible to plasma membrane constituents under specific conditions.

To address this issue, cortical neurons were surface biotinylated at 4°C prior to incubation at 37°C. Biotinylated surface proteins could either diffuse, or be internalized and their intermixing with BAD-LAMP positive compartments was evaluated at different time points by confocal microscopy. Biotinylated proteins were detected rapidly co-localizing with BAD-LAMP after 5 min of internalization. This significant overlapping distribution was decreased after 45 min, suggesting that BAD-LAMP containing organelles could represent a subset of early endocytic vesicles, rapidly accessible from the neuronal surface and serving as an intermediate step for the intracellular sorting of specific surface molecules present in developing neurons.

### BAD-LAMP sorting in transfected neurons

To further investigate distribution of BAD-LAMP, N- terminally FLAG and C-terminally GFP-tagged BAD-LAMP constructs were generated and their behaviour was monitored by microscopy in co-transfection experiments of cortical neurons.

Surprisingly, endogenous BAD-LAMP expression and domain organization were strongly inhibited in electroporated neurons. Nevertheless, transfected FLAG-tagged BAD-LAMP was found enriched in vesicles clustered in specific zones along the neurites.

Clearly, the tagged-protein is not addressed in conventional endo/lysosomes as judged by its lack of co-localization with LAMP2, internalized transferring or cholera toxin. FLAG-BAD-LAMP exact localization in the cell body was difficult to establish since its over-expression induced an accumulation of the molecule in the ER and Golgi apparatus. Surprisingly the C-terminally GFP-tagged, BAD-LAMP (BAD-GFP) was found accumulating in LAMP2-positive lysosomal compartments. BAD-LAMP cytoplasmic tail contains therefore a cryptic lysosomal retention motif, which is revealed by the addition of the GFP moiety. This observation also suggests that the cytoplasmic tail of BAD-LAMP is actively interacting with, or modified by, molecules which promote its sorting away from traditional endocytic compartments.

Co-localization of BAD-GFP and FLAG-tagged BAD-LAMP was observed in discrete vesicles in neurites, despite the fact that BAD-GFP was found mostly accumulating in large lysosomes in the cell body. This demonstrates that a small fraction of BAD-GFP can be sorted normally.

The internalization dynamics of BAD-LAMP was next evaluated by using the N-terminally FLAG-tagged construct and by monitoring FLAG antibody uptake after cold binding. The antibody was rapidly endocytosed after 5 min at 37°C. Inside the cell, it was detected in a compartment different from conventional endo/lysosomes as shown by the absence of co-localization with co-transfected BAD-GFP, LAMP2 and internalized cholera toxin. After 30 min of synchronous uptake, co-localization of the antibodies with BAD-LAMP-GFP and LAMP 2 indicated that BAD-LAMP can reach conventional endocytic compartments, after being internalized from the surface. Surprisingly, this co-localization was more evident in the more discrete LAMP2 positive organelles present in the neurites, than in the large lysosomes observed in the cell body.

The contribution of tyrosine 276 to BAD-LAMP trafficking was next investigated by introducing a mutational change to alanine at this position (Tyr276Ala). The FLAG-tagged mutant was also found accumulating in the ER and Golgi apparatus of transfected neurons. However, the fraction of the mutant exiting these organelles accumulated at the surface of the neurites in a manner very distinct from the normal molecule (wt), which was mostly found in intracellular vesicles. Similar results were obtained with a construct lacking the entire cytoplasmic tail of BAD-LAMP. Thus, tyrosine 276 is directly involved in intracellular addressing of BAD-LAMP and allows its internalization from the surface. FLAG antibody uptake after binding in the cold was performed in neurons expressing FLAG-BAD-LAMP Tyr276Ala. Transfected cells remained mostly antibody-decorated at the surface 30 min after warming-up at 37°C. Thus, BAD-LAMP is probably cycling between the plasma membrane and a subset of endocytic vesicles.

### BAD-LAMP recycles in HeLa cells.

In order to further dissect the molecular mechanisms governing BAD-LAMP endocytosis, the distribution and transport of transfected BAD-LAMP was studied in a cell type easy to manipulate, such as HeLa and mouse NIH 3T3 cells.

In HeLa cells, FLAG-BAD-LAMP was found at the cell surface and in internalized transferring containing vesicles distributed in the vicinity of the plasma membrane, while the Tyr276Ala mutant accumulated only at the cell surface (Figure 9A). No co-localization was found in LAMP1-positive late endosomes or lysosomes, nor with co-transfected DC-LAMP tagged with GFP, another lysosomal resident of the LAMP family (de Saint-Vis et al., 1998).

These observations were confirmed after Percoll density gradient subcellular fractionation of transfected HeLa cells **(****Figure 12****):** BAD-LAMP was mostly detected in the low density fractions of the gradient containing plasma membrane, ER and early endosomes, while it was absent from the high density fractions containing lysosomes as indicated by β-hexosaminidase activity.

Thus, most of transfected BAD-LAMP was found on the cell surface contrasting with transfected neurons in which BAD-LAMP mostly accumulated intracellularly, underlining the specificity of its sorting even when over-expressed.

Anti-FLAG antibody uptake in transfected cells saturated with FITC-transferrin (FITCTF), confirmed that BAD-LAMP could be internalized rapidly in sorting endosomes. Interestingly, 15 min after uptake BAD-LAMP was found present in transferrin positive recycling endosomes clustered around the microtubule organizing center, suggesting that BAD-LAMP could recycle to the plasma membrane after internalization. This hypothesis was supported by the poor co-localization of the antibody with LAMP1 after 45 min of uptake, indicating that the molecule does not reach efficiently late endocytic compartments. This underlines again a difference with neurons, in which the antibodies could be detected in discrete LAMP1 positive compartment 45 min after uptake.

The molecular mechanisms involved in BAD-LAMP endocytosis were next investigated.

Experiments performed in cells co-transfected with wild type GTPase dynamin II or dominant negative mutant A44K, indicated that BAD-LAMP internalization is mediated in a dynamin-dependent manner, since antibody internalization was abolished in cells expressing dynamin A44K.

In order to further define the endocytic pathway used by BAD-LAMP to enter the cell, an RNA inhibition approach was performed to reduce the expression of molecules involved in protein triage from the surface such as the clathrin adaptor AP-2 (Dugast et al., 2005; McCormick et al., 2005). Antibody uptake was monitored by immunostaining and FACS detection after binding at 4°C and internalization at 37°C.

Cells co-transfected with FLAG-BAD-LAMP and control RNAi plasmid showed rapid internalization of the antibody, while RNAi depletion of AP2 clearly inhibited BAD-LAMP internalization as well as transferrin uptake. In cells depleted for AP2, higher surface levels of BAD-LAMP were also constantly detected, suggesting that BAD-LAMP is internalized constantly through a dynamin/AP2 dependent endocytic pathway.

Interestingly monitoring of surface anti-flag antibody by FACS also indicated that the molecule was rapidly internalized between 5 and 7.5 min after warming up **(****Figure 13****).** Surface levels of antibodies then re-increased after 10min, to be diminished again but with a relatively slower internalization rate. These observations confirm that BAD-LAMP and associated antibodies constantly recycle to the plasma membrane with a relatively high efficiency.

### Discussion

BAD-LAMP sequence analysis clearly indicates that it represents a new member of the LAMP family. However its expression pattern and intracellular distribution are unconventional compared to other LAMP family members, which show a widespread expression and specifically accumulate in the lysosomes.

The inventors' observations on BAD-LAMP intracellular distribution are clearly indicative of a strong regulation of its trafficking in a subset of early endosomes.

The absence of co-localization with transferrin or synaptotagmin 1, as well as late endosomal markers such as LAMP1 suggests that these vesicles represent a distinct class of neuronal endosomes. The kinetics of biotinylated proteins and antibody uptake indicate that they can serve as sorting platforms, prior to transport to other organelles, which are positive for LAMP1, but only represent a minor fraction of the neuronal organelles containing LAMP1.

The inventors have shown that BAD-LAMP, through an interaction with its YKHM domain, requires dynamin and AP-2 to be internalized and sorted towards the early endocytic recycling pathway of transfected HeLa cells. LAMP1 has also been shown to require the AP-2 adaptor, but its sorting is directed towards lysosomes (Janvier and Bonifacino, 2005). Interestingly, modification of the BAD-LAMP C-terminal domain by GFP affects deeply its transport in neurons and demonstrates the existence of an active sorting pathway in these cells, which normally prevents the accumulation of BAD-LAMP in the lysosomes. The YKHM domain is a relatively weak consensus endosomal/lysosomal addressing signal (Bonifacino and Traub, 2003), although it is also found in CTLA-4, a molecule known to recycle upon activation of T cells (Linsley et al., 1996).

As suggested by its early endosomes distribution, it could be shown that BAD-LAMP also recycles in transfected HeLa cells. Whether this is the case in neurons remains to be further investigated, although it clearly indicates that the "YKHM" domain is not normally used as a lysosomal addressing signal.

One of the features of BAD-LAMP containing organelles is their clustered distribution. This distribution mirrors the organization of the different microdomains at the cell surface. Whether BAD-LAMP containing organelles participate in the maintenance of this organization within the neuritic plasma membrane remains to be definitely proven. Nevertheless their sensitivity to cholesterol depleting drugs suggests that microdomains and BAD-LAMP containing vesicles are functionally linked. Strikingly, the clustering of the BAD-LAMP containing vesicles is also defined by the distribution of the phosphorylated epitopes (SMI31) found on the microtubule associated protein MAP1B or Neurofilament H (Fischer and Romano-Clarke, 1990). MAP1 B in the cortex has been strongly implicated in synapse formation and function (Kawakami et al., 2003). Such a role has been functionally demonstrated in mice lacking the phosphorylated form of MAP1B specifically in the hippocampus, which show deficits in long term potentiation in the Schaeffer collaterals pathway (Zervas et al., 2005). It is conceivable that MAP1 B is implicated in the positioning and transport of BADLAMP vesicles at sites of postsynaptic densities on the dendrites of cortical neurons, and that this process could be essential for stabilization, function and plasticity of cortical synapses. This is also supported by the timing of BAD-LAMP expression.

Synaptogenesis in the cortex occurs in the postnatal animal with a peak between P10 and P15 to approach adult values (Micheva and Beaulieu, 1996). This increase in functional synapses in the cortex is strikingly mirrored by the expression of BADLAMP. Thus, it appears possible that BAD-LAMP is involved in the terminal maturation steps of defined cortical neuron populations. The transformation of a transient contact between two neurons into a stable and functional synapse requires major changes in the membrane composition of the respective neuronal surface areas.

Endocytic processes have been implicated in the regulation of synaptic function and plasticity in vertebrates (Vissel et al., 2001) and in drosophila (Dickman et al., 2006). For example, NMDA receptors are subject to constitutive (Roche et al., 2001) as well as agonist-induced (Vissel et al., 2001) internalization through clathrin mediated endocytosis. Interestingly, *in situ* hybridization for NMDAR1 resulted in strong cellular labeling in neurons of layers II/III, V and VI (Rudolf et al., 1996), resembling the pattern which the inventors found for BAD-LAMP in the postnatal cortex. The BADLAMP containing endocytic compartments could therefore play a regulatory role in these events by maintaining specific zones in the neuronal projections.

### Example 2

### Extracellular domain of human BAD-LAMP production and purification

The sequence encoding the extracellular domain of human BAD-LAMP (SEQ ID NO: 31) was inserted into a pSecTag2 vector (Invitrogen, #V900-20) between *Hind*III and *Xho*l restriction sites, and fused to a FLAG-tag encoding sequence to yield pSec-BAD.

HeLa 229 cells were transfected with pSec-BAD and cultured. After 4 days, the cell culture supernatant was taken and incubated overnight at 4°C with Ni-carrying beads (Ni-NTA, Quiagen). The beads are then taken and washed three times in a PBS / 12,5mM imidazole wash buffer. The extracellular domain is then eluted from the beads with a PBS / 250 mM imidazole elution buffer.

### Example 3

### Anti-BAD-LAMP monoclonal antibody production

### 1. Antibodies to the cytoplasmic part of BAD-LAMP

Mice and rats were immunized with a peptide of sequence KMTANQVQIPRDRSQYKHMC (SEQ ID NO: 28) which is present both in mice and human BAD-LAMP.

Animals were first injected intra-peritonealy (IP) with a mixture of 7.5 µg (mice) or 50 µg (rats) of peptide coupled to ovalbumine, by a SPdP or glutaraldehyde-based method (a mix of both), in 500 µL of water and 500 µL of complete Freund adjuvant (CFA).

Three other intra-peritoneal injections were performed every three weeks with identical mixtures, except for incomplete Freund adjuvant (IFA) in replacement of CFA.

Three weeks after the last intra-peritoneal injection, three daily intra-venous (IV) injections were performed with 20 µg of peptide in 200 µL by the caudal vein route (mice) or 50 µg of peptide in 200 µL by the penis (rats). The last day, animals were sacrificed to get spleen cells.

### 2. Antibodies to the extra-cellular part of BAD-LAMP

A vaccination scheme similar to that used for the generation of antibodies to the cytoplasmic part of human BAD-LAMP was devised except that the peptide was replaced by HeLa 229 transfected with a complete human BAD-LAMP (SEQ ID NO: 2) expression system. This construct is characterized by a FLAG tag (Sigma) fused with BAD-LAMP at the N-terminal end. Briefly, 10⁶ (mice) or 10⁷ (rats) 16-hours transfected HeLa 229 cells were used for the IP injections, and 10⁶ (mice and rats) 16-hours transfected HeLa 229 cells were used for the IV injections.

### 3. Hybridoma generation

Spleen cells from immunized rats and mice were fused with X63 myeloma cells Kohler & Milstein (1975) *Nature* **256**:495 according to methods well known in the art.

Hybridoma clones yielding antibodies to the cytoplasmic part of BAD-LAMP were screened by ELISA with the Aquabind kit (Iwaki Glass Co. Ltd., Tokyo, Japan) following the manufacturer's instructions. Briefly, the immunoreactivity of conditioned media from the hybridoma clones towards the immunizing peptide was assessed by ELISA. Immunoreactive clones were then selected and went through a second round of cloning and immunoreactive selection. The clones the conditioned medium of which was shown to be immunoreactive were then expanded.

4 anti-BAD-LAMP clones targeting the cytoplasmic domain of mouse and human BAD-LAMP were thus recovered including mBAD 14.2 which has been deposited at the CNCM (Institut Pasteur, Paris, France) under the Budapest Treaty on October 19, 2006 with reference number I-3684.

The conditioned media of clones yielding antibodies to the extracellular part of BAD-LAMP were screened by FACS using CHO cells expressing the FLAG-BAD-LAMP produced as described in section 2. Immunoreactive clones were then selected and went through a second round of cloning and immunoreactive selection. The clones the conditioned medium of which was shown to be immunoreactive were then expanded.

1 rat anti-human BAD-LAMP clone targeting the extracellular domain of human BAD-LAMP was thus recovered, hBAD 15F8, which has been deposited at the CNCM (Institut Pasteur, Paris, France) under the Budapest Treaty on October 19, 2006 with reference number 1-3679.

### Example 4

### Expression of human BAD-LAMP in human plasmacytoid dendritic cells

In addition to the northern blot described in **Example 1**, a nested-PCR approach applied to human spleen mRNAs, a slight expression of human BAD-LAMP, probably restricted to a small population of cells of the immune system, was detected.

Immunofluorescence experiments applied to spleen and amygdale sections confirmed that human BAD-LAMP was expressed in a particular set of cells of the immune system, in particular CD123+ CD4+ cells. The association of CD123 and CD4 expression is specific of plasmacytoid dendritic cells.

This particular pattern of expression was further confirmed by sorting plasmacytoid dendritic cells from peripheral blood leukocytes using BDCA4 as a plasmacytoid dendritic cell-specific marker, essentially as described by Dzionek et al. (2000) J. immunol. 165:6037-6046. Specific human BAD-LAMP mRNA expression in the purified plasmacytoid dendritic cells, with respect to peripheral blood monocytes, was then confirmed by RT-PCR, essentially as described by Bania et al. (2003) Proc. Natl. Acad. Sci. USA 100:6664-6669 **(****Figure 16****).** In addition, expression of the human BAD-LAMP protein, using anti-BAD-LAMP monoclonal antibodies, was evidenced in plasmacytoid dendritic cells but not in peripheral blood monocytes **(****Figure 15****).**

Besides, immunofluorescent marking of human BAD-LAMP, carried out essentially as described by Lelouard et al. (2002) Nature 417:177-182 with anti-BAD-LAMP monoclonal antibodies, showed that it was expressed in the cytoplasmic membrane and in various intracellular vesicular compartments.

### References

Andrejewski N, Punnonen EL, Guhde G, Tanaka Y, Lullmann-Rauch R, Hartmann D, von Figura K, Saftig P (1999) Normal lysosomal morphology and function in LAMP-1-deficient mice. J. Biol. Chem. 274:12692-12701.
Bonifacino JS, Traub LM (2003) Signals for sorting of transmembrane proteins to endosomes and lysosomes. Annu Rev Biochem 72:395-447.
Cappello F, Gatti E, Camossetto V, David A, Lelouard H, Pierre P (2004) Cystatin F is secreted, but artificial modification of its C-terminus can induce its endocytic targeting. Exp Cell Res 297:607-618.
Coco S, Raposo G, Martinez S, Fontaine JJ, Takamori S, Zahraoui A, Jahn R, Matteoli M, Louvard D, Galli T (1999) Subcellular localization of tetanus neurotoxin-insensitive vesicle-associated membrane protein (VAMP)/VAMP7 in neuronal cells: evidence for a novel membrane compartment. J Neurosci 19:9803-9812.
de Saint-Vis B, Vincent J, Vandenabeele S, Vanbervliet B, Pin JJ, Ait-Yahia S, Patel S, Mattei MG, Banchereau J, Zurawski S, Davoust J, Caux C, Lebecque S (1998) A novel lysosome-associated membrane glycoprotein, DC-LAMP, induced upon DC maturation, is transiently expressed in MHC class II compartment. Immunity 9:325-336.
Dehmelt L, Halpain S (2004) Actin and microtubules in neurite initiation: are MAPs the missing link? J Neurobiol 58:18-33.
Del Rio JA, Gonzalez-Billault C, Urena JM, Jimenez EM, Barallobre MJ, Pascual M, Pujadas L, Simo S, La Torre A, Wandosell F, Avila J, Soriano E (2004) MAP1 B is required for Netrin 1 signaling in neuronal migration and axonal guidance. Curr Biol 14:840-850.
Dickman DK, Lu Z, Meinertzhagen IA, Schwarz TL (2006) Altered synaptic development and active zone spacing in endocytosis mutants. Curr Biol 16:591-598.
Dugast M, Toussaint H, Dousset C, Benaroch P (2005) AP2 clathrin adaptor complex, but not AP1, controls the access of the major histocompatibility complex (MHC) class II to endosomes. J Biol Chem 280:19656-19664.
Eskelinen EL, Tanaka Y, Saftig P (2003) At the acidic edge: emerging functions for lysosomal membrane proteins. Trends Cell Biol 13 :137-145.
Eskelinen EL, IIIert AL, Tanaka Y, Schwarzmann G, Blanz J, Von Figura K, Saftig P (2002) Role of LAMP-2 in lysosome biogenesis and autophagy. Mol Biol Cell 13:3355-3368.
Fischer I, Romano-Clarke G (1990) Changes in microtubule-associated protein MAP1B phosphorylation during rat brain development. J Neurochem 55:328-333.
Gruenberg J, Stenmark H (2004) The biogenesis of multivesicular endosomes. Nat Rev Mol Cell Biol 5:317-323.
Hirling H, Steiner P, Chaperon C, Marsault R, Regazzi R, Catsicas S (2000) Syntaxin 13 is a developmentally regulated SNARE involved in neurite outgrowth and endosomal trafficking. Eur J Neurosci 12:1913-1923.
Janvier K, Bonifacino JS (2005) Role of the endocytic machinery in the sorting of lysosome-associated membrane proteins. Mol Biol Cell 16:4231-4242.
Kawakami S, Muramoto K, Ichikawa M, Kuroda Y (2003) Localization of microtubule-associated protein (MAP) 1 B in the postsynaptic densities of the rat cerebral cortex. Cell Mol Neurobiol 23:887-894.
Kennedy MJ, Ehlers MD (2006) Organelles and trafficking machinery for postsynaptic plasticity. Annu Rev Neurosci 29:325-362.
Lin JH, Saito T, Anderson DJ, Lance-Jones C, Jessell TM, Arber S (1998) Functionally related motor neuron pool and muscle sensory afferent subtypes defined by coordinate ETS gene expression. Cell 95:393-407.
Linsley PS, Bradshaw J, Greene J, Peach R, Bennett KL, Mittler RS (1996) Intracellular trafficking of CTLA-4 and focal localization towards sites of TCR engagement. Immunity 4:535-543.
Madore N, Smith KL, Graham CH, Jen A, Brady K, Hall S, Morris R (1999) Functionally different GPI proteins are organized in different domains on the neuronal surface. Embo J 18:6917-6926.
Maxfield FR, Tabas I (2005) Role of cholesterol and lipid organization in disease. Nature 438:612-621.
McCormick PJ, Martina JA, Bonifacino JS (2005) Involvement of clathrin and AP-2 in the trafficking of MHC class II molecules to antigen-processing compartments. Proc Natl Acad Sci U S A 102:7910-7915.
Micheva KD, Beaulieu C (1996) Quantitative aspects of synaptogenesis in the rat barrel field cortex with special reference to GABA circuitry. J Comp Neurol 373:340-354.
Rice DS, Curran T (1999) Mutant mice with scrambled brains: understanding the signaling pathways that control cell positioning in the CNS. Genes Dev 13:2758-2773.
Roche KW, Standley S, McCallum J, Dune Ly C, Ehlers MD, Wenthold RJ (2001) Molecular determinants of NMDA receptor internalization. Nat Neurosci 4:794-802.
Rudolf GD, Cronin CA, Landwehrmeyer GB, Standaert DG, Penney JB, Jr., Young AB (1996) Expression of N-methyl-D-aspartate glutamate receptor subunits in the prefrontal cortex of the rat. Neuroscience 73:417-427.
Saftig P, Tanaka Y, Lullmann-Rauch R, von Figura K (2001) Disease model: LAMP-2 enlightens Danon disease. Trends Mol Med 7:37-39.
Santuccione A, Sytnyk V, Leshchyns'ka I, Schachner M (2005) Prion protein recruits its neuronal receptor NCAM to lipid rafts to activate p59fyn and to enhance neurite outgrowth. J Cell Biol 169:341-354.
Shikano S, Bonkobara M, Zukas PK, Ariizumi K (2001) Molecular cloning of a dendritic cell-associated transmembrane protein, DC-HIL, that promotes RGDdependent adhesion of endothelial cells through recognition of heparan sulphate proteoglycans. J Biol Chem 276:8125-8134.
Sunyach C, Jen A, Deng J, Fitzgerald KT, Frobert Y, Grassi J, McCaffrey MW, Morris R (2003) The mechanism of internalization of glycosylphosphatidylinositolanchored prion protein. Embo J 22:3591-3601.
Sytnyk V, Leshchyns'ka I, Dityatev A, Schachner M (2004) Trans-Golgi network delivery of synaptic proteins in synaptogenesis. J Cell Sci 117:381-388.
Sytnyk V, Leshchyns'ka I, Delling M, Dityateva G, Dityatev A, Schachner M (2002) Neural cell adhesion molecule promotes accumulation of TGN organelles at sites of neuron-to-neuron contacts. J Cell Biol 159:649-661.
Tanaka Y, Guhde G, Suter A, Eskelinen EL, Hartmann D, Lullmann-Rauch R, Janssen PM, Blanz J, von Figura K, Saftig P (2000) Accumulation of autophagic vacuoles and cardiomyopathy in LAMP-2-deficient mice. Nature 406:902-906.
Tiveron MC, Hirsch MR, Brunet JF (1996) The expression pattern of the transcription factor Phox2 delineates synaptic pathways of the autonomic nervous system. J Neurosci 16:7649-7660.
Vissel B, Krupp JJ, Heinemann SF, Westbrook GL (2001) A use-dependent tyrosine dephosphorylation of NMDA receptors is independent of ion flux. Nat Neurosci 4:587-596.
Zervas M, Opitz T, Edelmann W, Wainer B, Kucherlapati R, Stanton PK (2005) Impaired hippocampal long-term potentiation in microtubule-associated protein 1 B-deficient mice. J Neurosci Res 82 :83-92.
Zimmer C, Tiveron MC, Bodmer R, Cremer H (2004) Dynamics of Cux2 expression suggests that an early pool of SVZ precursors is fated to become upper cortical layer neurons. Cereb Cortex 14:1408-1420.

## Claims

1. An isolated protein comprising or consisting of:
- a sequence represented by SEQ ID NO: 2 *(Homo sapiens* BAD-LAMP), or
- a homologous sequence which presents at least 20% identity to SEQ ID NO: 2, provided that the homologous sequence represents a protein having an activity similar to that of *Homo sapiens* BAD-LAMP.

2. An isolated protein according to claim 1, represented by a sequence selected from the group consisting of SEQ ID NO: 2, SEQ ID NO: 4 *(Mus musculus* BAD-LAMP), SEQ ID NO: 6 *(Rattus norvegicus* BAD-LAMP), SEQ ID NO: 8 *(Bos taurus* BAD-LAMP), SEQ ID NO: 10 *(Pan troglodytes* BAD-LAMP), SEQ ID NO: 12 *(Monodelphis domestica* BAD-LAMP), SEQ ID NO: 14 (*Xenopus tropicalis* BAD-LAMP), SEQ ID NO: 16 *(Gallus gallus* BAD-LAMP), SEQ ID NO: 18 *(Anopheles gambiae* BAD-LAMP), SEQ ID NO: 20 *(Tetraodon viridis* BAD-LAMP), SEQ ID NO: 22 *(Takifugu rubripes* BAD-LAMP) and SEQ ID NO: 24 *(Drosophila melanogaster* BAD-LAMP).

3. A fragment of an isolated protein consisting of:
- a sequence represented by SEQ ID NO: 2, or
- a homologous sequence which presents at least 20% identity to SEQ ID NO: 2, provided that the homologous sequence represents a protein having an activity similar to that of *Homo sapiens* BAD-LAMP,
or of an isolated protein as defined in claim 2,
wherein said fragment comprises at least 10 amino acids.

4. An isolated nucleic acid comprising or consisting of:
- a nucleotide sequence encoding an isolated protein as defined in claim 1 or 2, or a fragment as defined in claim 3, or
- a homologous nucleotide sequence acid which presents at least 80% homology to said nucleotide sequence encoding an isolated protein as defined in claim 1 or 2, provided that homologous sequence encodes a protein presenting an activity similar to that of *Homo sapiens* BAD-LAMP; or
- a mutant nucleotide sequence which derives from said nucleotide sequence encoding an isolated protein as defined in claim 1 or 2 by insertion, deletion or substitution of at least one nucleotide, provided that mutant sequence encodes a protein presenting an activity similar to that of *Homo sapiens* BAD-LAMP; or
- a hybridizing nucleotide sequence which hybridizes under stringent conditions with said nucleotide sequence encoding an isolated protein as defined in claim 1 or 2, provided that the complementary sequence of said hybridizing nucleotide sequence encodes a protein presenting an activity similar to that of *Homo sapiens* BAD-LAMP; or
- the complementary sequences of said isolated protein-encoding nucleotide sequence, homologous nucleotide sequence, mutant nucleotide sequence and hybridizing nucleotide sequence.

5. An isolated nucleic acid according to claim 4, represented by a sequence selected from the group consisting of SEQ ID NO: 1, SEQ ID NO: 3 *(Mus musculus* BAD-LAMP), SEQ ID NO: 5 *(Rattus norvegicus* BAD-LAMP), SEQ ID NO: 7 *(Bos taurus* BAD-LAMP), SEQ ID NO: 9 *(Pan troglodytes* BAD-LAMP), SEQ ID NO: 11 *(Monodelphis domestica* BAD-LAMP), SEQ ID NO: 13 (*Xenopus tropicalis* BAD-LAMP), SEQ ID NO: 15 *(Gallus gallus* BAD-LAMP), SEQ ID NO: 17 *(Anopheles gambiae* BAD-LAMP), SEQ ID NO: 19 (*Tetraodon viridis* BAD-LAMP), SEQ ID NO: 21 (*Takifugu rubripes* BAD-LAMP) and SEQ ID NO: 23 *(Drosophila melanogaster* BAD-LAMP).

6. A ligand comprising at least one binding moiety, wherein said at least one binding moiety is specific for an isolated protein as defined in claim 1 or 2, for a fragment as defined in claim 3, or for a protein encoded by an isolated nucleic acid as defined in claim 4 or 5.

7. A ligand according to claim 6, wherein the at least one binding moiety is specific for a protein represented by a sequence selected from the group consisting of SEQ ID NO: 2, SEQ ID NO: 4, SEQ ID NO: 6, SEQ ID NO: 8, and SEQ ID NO: 10.

8. A ligand according to claim 6 or 7, wherein the at least one binding moiety is selected from the group consisting of an antibody, an antigen-specific antibody fragment, a scFv, or an aptamer.

9. A ligand according to any of claims 6 to 8, wherein the at least one binding moiety is a monoclonal antibody.

10. A ligand according to claim 9, wherein the monoclonal antibody is produced by one of the following hybridomas:
- a hybridoma deposited at the Collection Nationale de Cultures de Microorganismes (CNCM, Paris, France) under the Budapest Treaty on October 19, 2006 with accession number I-3679, or
- a hybridoma deposited at the Collection Nationale de Cultures de Microorganismes (CNCM, Paris, France) under the Budapest Treaty on October 19, 2006 with accession number I-3684.

11. A ligand according to any of claims 6 to 10, further comprising at least one detectable marker.

12. A ligand according to claim 11, wherein the at least one detectable marker is selected from the group consisting of an enzyme, a chromophore, or a fluorophore.

13. A ligand according to claims 11 or 12, consisting of at least one binding moiety linked to at least one detectable marker.

14. A ligand according to any of claims 6 to 10, consisting of at least one binding moiety.

15. The use of a ligand according to any of claims 6 to 14, for detecting human plasmacytoid dendritic cells in a biological sample and/or for purifying human plasmacytoid dendritic cells from a biological sample.

16. The use of a nucleic acid as defined in claim 4, which is liable to bind under stringent conditions to a RNA encoding a protein represented by SEQ ID NO: 2, for detecting human plasmacytoid dendritic cells in a biological sample.

17. A kit for the detection and/or the purification of human plasmacytoid dendritic cells comprising:
- a ligand as defined in any of claims 6 to 14,
- a control consisting of an isolated protein as defined in claims 1 or 2, or a fragment as defined in claim 3,
- at least one suitable buffer,

18. A method for detecting human plasmacytoid dendritic cells in a biological sample, comprising a step of detecting the expression of a protein as defined in claim 1 in cells of the biological sample, whereby the cells in which the expression of a protein as defined in claim 1 is detected are human plasmacytoid dendritic cells.

19. A method for detecting human plasmacytoid dendritic cells according to claim 18, comprising the following steps:
- contacting the biological sample with at least one ligand according to any of claims 6 to 14 to form a mixture,
- incubating the mixture in conditions allowing the formation of complexes between the ligand and a protein as defined in claim 1,
- detecting the complexes formed between the ligand and the protein,
whereby the presence of human plasmacytoid dendritic cells is detected.

20. A method for purifying human plasmacytoid dendritic cells from a biological sample, comprising the following steps:
- detecting the expression of a protein as defined in claim 1 in cells of the biological sample,
- selecting cells in which the expression the protein is detected,
whereby human plasmacytoid dendritic cells are purified.

21. A method for purifying human plasmacytoid dendritic cells according to claim 20, comprising the following steps:
- contacting a biological sample with at least one ligand according to any of claims 6 to 14 to form a mixture,
- incubating the mixture in conditions allowing the formation of complexes between the ligand and a protein as defined in claim 1,
- separating the complexes between the ligand and the protein from the rest of the mixture,
whereby human plasmacytoid dendritic cells are purified.

22. An *in vitro* method for diagnosing the presence of a tumor in an individual, comprising a step of detecting the expression of an isolated protein as defined in claim 1 in cells of a tissue of the individual suspected of harbouring a tumor, wherein the expression of the isolated protein is indicative of the presence of a tumor in said tissue.

23. An *in vitro* method, for prognosing the evolution of a tumor or for evaluating the risk of metastasis of a tumor in an individual, comprising a step of detecting the expression of an isolated protein as defined in claim 1 in cells of a tissue harbouring the tumor, wherein the expression of the isolated protein is indicative of a growth of the tumor or of an increased risk of metastasis of the tumor, as compared to tumors which are not included in tissues which cells express an isolated protein as defined in claim 1.


SEQUENCE LISTING

<110>  INSERM

<120>  LYSOSOMAL ASSOCIATED MEMBRANE PROTEIN (LAMP) - LIKE POLYPEPTIDE,
       LIGANDS TO THE SAME, AND USE OF THE SAME IN THE FRAME OF
       DETECTION AND PURIFICATION OF HUMAN PLASMACYTOID DENDRITIC CELLS

<130>  BET 06P0423

<160>  31

<170>  PatentIn version 3.2

<210>  1
<211>  843
<212>  DNA
<213>  Homo sapiens

<220>
<221>  CDS
<222>  (1)..(843)

<400>  1
atg gat ctc caa gga aga ggg gtc ccc agc atc gac aga ctt cga gtt      48
Met Asp Leu Gln Gly Arg Gly Val Pro Ser Ile Asp Arg Leu Arg Val
1               5                   10                  15

ctc ctg atg ttg ttc cat aca atg gct caa atc atg gca gaa caa gaa      96
Leu Leu Met Leu Phe His Thr Met Ala Gln Ile Met Ala Glu Gln Glu
            20                  25                  30

gtg gaa aat ctc tca ggc ctt tcc act aac cct gaa aaa gat ata ttt      144
Val Glu Asn Leu Ser Gly Leu Ser Thr Asn Pro Glu Lys Asp Ile Phe
        35                  40                  45

gtg gtg cgg gaa aat ggg acg acg tgt ctc atg gca gag ttt gca gcc      192
Val Val Arg Glu Asn Gly Thr Thr Cys Leu Met Ala Glu Phe Ala Ala
    50                  55                  60

aaa ttt att gta cct tat gat gtg tgg gcc agc aac tac gta gat ctg      240
Lys Phe Ile Val Pro Tyr Asp Val Trp Ala Ser Asn Tyr Val Asp Leu
65                  70                  75                  80

atc aca gaa cag gcc gat atc gca ttg acc cgg gga gct gag gtg aag      288
Ile Thr Glu Gln Ala Asp Ile Ala Leu Thr Arg Gly Ala Glu Val Lys
                85                  90                  95

ggc cgc tgt ggc cac agc cag tcg gag ctg caa gtg ttc tgg gtg gat      336
Gly Arg Cys Gly His Ser Gln Ser Glu Leu Gln Val Phe Trp Val Asp
            100                 105                 110

cgc gca tat gca ctc aaa atg ctc ttt gta aag gaa agc cac aac atg      384
Arg Ala Tyr Ala Leu Lys Met Leu Phe Val Lys Glu Ser His Asn Met
        115                 120                 125

tcc aag gga cct gag gcg act tgg agg ctg agc aaa gtg cag ttt gtc      432
Ser Lys Gly Pro Glu Ala Thr Trp Arg Leu Ser Lys Val Gln Phe Val
    130                 135                 140

tac gac tcc tcg gag aaa acc cac ttc aaa gac gca gtc agt gct ggg 480
Tyr Asp Ser Ser Glu Lys Thr His Phe Lys Asp Ala Val Ser Ala Gly
145                 150                 155                 160

aag cac aca gcc aac tcg cac cac ctc tct gcc ttg gtc acc ccc gct 528
Lys His Thr Ala Asn Ser His His Leu Ser Ala Leu Val Thr Pro Ala
                165                 170                 175

ggg aag tcc tat gag tgt caa gct caa caa acc att tca ctg gcc tct 576
Gly Lys Ser Tyr Glu Cys Gln Ala Gln Gln Thr Ile Ser Leu Ala Ser
            180                 185                 190

agt gat ccg cag aag acg gtc acc atg atc ctg tct gcg gtc cac atc 624
Ser Asp Pro Gln Lys Thr Val Thr Met Ile Leu Ser Ala Val His Ile
        195                 200                 205

caa cct ttt gac att atc tca gat ttt gtc ttc agt gaa gag cat aaa 672
Gln Pro Phe Asp Ile Ile Ser Asp Phe Val Phe Ser Glu Glu His Lys
    210                 215                 220

tgc cca gtg gat gag cgg gag caa ctg gaa gaa acc ttg ccc ctg att 720
Cys Pro Val Asp Glu Arg Glu Gln Leu Glu Glu Thr Leu Pro Leu Ile
225                 230                 235                 240

ttg ggg ctc atc ttg ggc ctc gtc atc atg gta aca ctc gcg att tac 768
Leu Gly Leu Ile Leu Gly Leu Val Ile Met Val Thr Leu Ala Ile Tyr
                245                 250                 255

cac gtc cac cac aaa atg act gcc aac cag gtg cag atc cct cgg gac 816
His Val His His Lys Met Thr Ala Asn Gln Val Gln Ile Pro Arg Asp
            260                 265                 270

aga tcc cag tat aag cac atg ggc tag 843
Arg Ser Gln Tyr Lys His Met Gly
        275                 280

<210> 2
<211> 280
<212> PRT
<213> Homo sapiens

<400> 2

Met Asp Leu Gln Gly Arg Gly Val Pro Ser Ile Asp Arg Leu Arg Val
1               5                   10                  15

Leu Leu Met Leu Phe His Thr Met Ala Gln Ile Met Ala Glu Gln Glu
            20                  25                  30

Val Glu Asn Leu Ser Gly Leu Ser Thr Asn Pro Glu Lys Asp Ile Phe
        35                  40                  45

Val Val Arg Glu Asn Gly Thr Thr Cys Leu Met Ala Glu Phe Ala Ala
    50                  55                  60

Lys Phe Ile Val Pro Tyr Asp Val Trp Ala Ser Asn Tyr Val Asp Leu

65                  70                  75                  80

Ile Thr Glu Gln Ala Asp Ile Ala Leu Thr Arg Gly Ala Glu Val Lys
                85                  90                  95

Gly Arg Cys Gly His Ser Gln Ser Glu Leu Gln Val Phe Trp Val Asp
            100                 105                 110

Arg Ala Tyr Ala Leu Lys Met Leu Phe Val Lys Glu Ser His Asn Met
        115                 120                 125

Ser Lys Gly Pro Glu Ala Thr Trp Arg Leu Ser Lys Val Gln Phe Val
    130                 135                 140

Tyr Asp Ser Ser Glu Lys Thr His Phe Lys Asp Ala Val Ser Ala Gly
145                 150                 155                 160

Lys His Thr Ala Asn Ser His His Leu Ser Ala Leu Val Thr Pro Ala
                165                 170                 175

Gly Lys Ser Tyr Glu Cys Gln Ala Gln Gln Thr Ile Ser Leu Ala Ser
            180                 185                 190

Ser Asp Pro Gln Lys Thr Val Thr Met Ile Leu Ser Ala Val His Ile
        195                 200                 205

Gln Pro Phe Asp Ile Ile Ser Asp Phe Val Phe Ser Glu Glu His Lys
    210                 215                 220

Cys Pro Val Asp Glu Arg Glu Gln Leu Glu Glu Thr Leu Pro Leu Ile
225                 230                 235                 240

Leu Gly Leu Ile Leu Gly Leu Val Ile Met Val Thr Leu Ala Ile Tyr
                245                 250                 255

His Val His His Lys Met Thr Ala Asn Gln Val Gln Ile Pro Arg Asp
            260                 265                 270

Arg Ser Gln Tyr Lys His Met Gly
        275                 280

<210>  3
<211>  843
<212>  DNA
<213>  Mus musculus

<220>
<221> CDS
<222> (1)..(843)

<400> 3
atg gat ctc cga gta aga acc ctt ctt ggt gga gac aga ctt cgg att 48
Met Asp Leu Arg Val Arg Thr Leu Leu Gly Gly Asp Arg Leu Arg Ile
1               5                   10                  15

ctc cta atg ttc ttc cat gta atg gtc caa acc gtg gca gaa caa gaa 96
Leu Leu Met Phe Phe His Val Met Val Gln Thr Val Ala Glu Gln Glu
            20                  25                  30

gtg gaa aat ctt tcg ggc ctt tcc acg aac ccc gag aaa gac ata ttt 144
Val Glu Asn Leu Ser Gly Leu Ser Thr Asn Pro Glu Lys Asp Ile Phe
        35                  40                  45

gtg gtg cgg gaa aat ggg acg acg tgt ctc atg gca gag ttt gca gcc 192
Val Val Arg Glu Asn Gly Thr Thr Cys Leu Met Ala Glu Phe Ala Ala
    50                  55                  60

aaa ttt att gta cct tat gat gtg tgg gcc agc aat tat gtg gat ctg 240
Lys Phe Ile Val Pro Tyr Asp Val Trp Ala Ser Asn Tyr Val Asp Leu
65                  70                  75                  80

atc aca gaa cag gct gag atc tcc ttg acc cgg gga gct gag gtg aag 288
Ile Thr Glu Gln Ala Glu Ile Ser Leu Thr Arg Gly Ala Glu Val Lys
                85                  90                  95

ggc cac tgt ggc cac aac gag tcg gag ctg gag gtg ttc tgg gta gat 336
Gly His Cys Gly His Asn Glu Ser Glu Leu Glu Val Phe Trp Val Asp
            100                 105                 110

cac gcc tac aca ctc aga atg ctc ttt gta aag gaa agt cac aac act 384
His Ala Tyr Thr Leu Arg Met Leu Phe Val Lys Glu Ser His Asn Thr
        115                 120                 125

tcc aaa gga cca gag gcg act tgg aat ctg aac aag gtg cat ttt gtc 432
Ser Lys Gly Pro Glu Ala Thr Trp Asn Leu Asn Lys Val His Phe Val
    130                 135                 140

tat gat tcc tcg gag aag act cac ttt aaa gcc cct gtg aag gtt aat 480
Tyr Asp Ser Ser Glu Lys Thr His Phe Lys Ala Pro Val Lys Val Asn
145                 150                 155                 160

aag tac atc gcc agc tct cat cac ctc tct gcc ttg gtc acc cca gct 528
Lys Tyr Ile Ala Ser Ser His His Leu Ser Ala Leu Val Thr Pro Ala
                165                 170                 175

ggg atg tcc tat gag tgt cag gct cag cag acc att tct ctg gcc tcc 576
Gly Met Ser Tyr Glu Cys Gln Ala Gln Gln Thr Ile Ser Leu Ala Ser
            180                 185                 190

agt gac cct cag aag act gtc acc atg atc ctg tcc gca gtg cac atc 624
Ser Asp Pro Gln Lys Thr Val Thr Met Ile Leu Ser Ala Val His Ile
        195                 200                 205

cag ccc ttt gat atc atc tct gac ttt gtc ttc agt gaa gaa cat aaa 672
Gln Pro Phe Asp Ile Ile Ser Asp Phe Val Phe Ser Glu Glu His Lys
    210                 215                 220

tgt cca gtg gat gag caa gag cag ttg gaa gag acc ctg ccc ctg atc          720
Cys Pro Val Asp Glu Gln Glu Gln Leu Glu Glu Thr Leu Pro Leu Ile
225                 230                 235                 240

ctg ggc ctc atc ttg ggc ctt gtc att gtc ata aca ctt gtg atc tac          768
Leu Gly Leu Ile Leu Gly Leu Val Ile Val Ile Thr Leu Val Ile Tyr
                245                 250                 255

cac atc cac cat aaa atg act gcc aat caa gtg cag atc ccc agg gac          816
His Ile His His Lys Met Thr Ala Asn Gln Val Gln Ile Pro Arg Asp
            260                 265                 270

cgt tcc cag tac aag cac atg ggc tag                                      843
Arg Ser Gln Tyr Lys His Met Gly
        275                 280

<210>  4
<211>  280
<212>  PRT
<213>  Mus musculus

<400>  4

Met Asp Leu Arg Val Arg Thr Leu Leu Gly Gly Asp Arg Leu Arg Ile
1               5                   10                  15

Leu Leu Met Phe Phe His Val Met Val Gln Thr Val Ala Glu Gln Glu
            20                  25                  30

Val Glu Asn Leu Ser Gly Leu Ser Thr Asn Pro Glu Lys Asp Ile Phe
        35                  40                  45

Val Val Arg Glu Asn Gly Thr Thr Cys Leu Met Ala Glu Phe Ala Ala
    50                  55                  60

Lys Phe Ile Val Pro Tyr Asp Val Trp Ala Ser Asn Tyr Val Asp Leu
65                  70                  75                  80

Ile Thr Glu Gln Ala Glu Ile Ser Leu Thr Arg Gly Ala Glu Val Lys
                85                  90                  95

Gly His Cys Gly His Asn Glu Ser Glu Leu Glu Val Phe Trp Val Asp
            100                 105                 110

His Ala Tyr Thr Leu Arg Met Leu Phe Val Lys Glu Ser His Asn Thr
        115                 120                 125

Ser Lys Gly Pro Glu Ala Thr Trp Asn Leu Asn Lys Val His Phe Val
    130                 135                 140

Tyr Asp Ser Ser Glu Lys Thr His Phe Lys Ala Pro Val Lys Val Asn

145                 150                 155                 160

Lys Tyr Ile Ala Ser Ser His His Leu Ser Ala Leu Val Thr Pro Ala
                165                 170                 175

Gly Met Ser Tyr Glu Cys Gln Ala Gln Gln Thr Ile Ser Leu Ala Ser
            180                 185                 190

Ser Asp Pro Gln Lys Thr Val Thr Met Ile Leu Ser Ala Val His Ile
        195                 200                 205

Gln Pro Phe Asp Ile Ile Ser Asp Phe Val Phe Ser Glu Glu His Lys
    210                 215                 220

Cys Pro Val Asp Glu Gln Glu Gln Leu Glu Glu Thr Leu Pro Leu Ile
225                 230                 235                 240

Leu Gly Leu Ile Leu Gly Leu Val Ile Val Ile Thr Leu Val Ile Tyr
                245                 250                 255

His Ile His His Lys Met Thr Ala Asn Gln Val Gln Ile Pro Arg Asp
            260                 265                 270

Arg Ser Gln Tyr Lys His Met Gly
        275                 280

<210>  5
<211>  843
<212>  DNA
<213>  Rattus norvegicus

<220>
<221>  CDS
<222>  (1)..(843)

<400>  5
atg gat cta cga gga aga gcc ctt ctc ggt gga gac aga ctt cgg att        48
Met Asp Leu Arg Gly Arg Ala Leu Leu Gly Gly Asp Arg Leu Arg Ile
1               5                   10                  15

ctc cta atg ttc ttc cat gca atg gcc caa acc gtg gca gaa caa gaa        96
Leu Leu Met Phe Phe His Ala Met Ala Gln Thr Val Ala Glu Gln Glu
            20                  25                  30

gtg gaa aat ctt tcg ggc ctt tcc acg aac ccg gag aaa gac ata ttt       144
Val Glu Asn Leu Ser Gly Leu Ser Thr Asn Pro Glu Lys Asp Ile Phe
        35                  40                  45

gtg gtg cgg gaa aat ggg acg acg tgt ctc atg gca gag ttt gca gcc       192
Val Val Arg Glu Asn Gly Thr Thr Cys Leu Met Ala Glu Phe Ala Ala
    50                  55                  60

aaa ttt att gta cct tat gat gtg tgg gcc agc aat tat gtg gat ctg 240
Lys Phe Ile Val Pro Tyr Asp Val Trp Ala Ser Asn Tyr Val Asp Leu
65                  70                  75                  80

atc aca gag cag gct gag atc tcc ttg acc cgg gga gct gag gtg aag 288
Ile Thr Glu Gln Ala Glu Ile Ser Leu Thr Arg Gly Ala Glu Val Lys
                85                  90                  95

ggc cgc tgt ggt cac aac gag tcg gag ctg cag gtg ttc tgg gta gat 336
Gly Arg Cys Gly His Asn Glu Ser Glu Leu Gln Val Phe Trp Val Asp
            100                 105                 110

cgc gcc tac aca ctc aaa atg ctc ttt gta aag gaa agt cac aac act 384
Arg Ala Tyr Thr Leu Lys Met Leu Phe Val Lys Glu Ser His Asn Thr
        115                 120                 125

tcc aaa gga cta gag gcg act tgg aag ctg agc aag gtg cag ttt gtc 432
Ser Lys Gly Leu Glu Ala Thr Trp Lys Leu Ser Lys Val Gln Phe Val
    130                 135                 140

tat gat tcc tca gag aag acc cac ttt aaa gat gct gtg agt gct ggg 480
Tyr Asp Ser Ser Glu Lys Thr His Phe Lys Asp Ala Val Ser Ala Gly
145                 150                 155                 160

aag cat acc gcc aac tct cac cac ctc tct gcc ttg gtg acc cca gct 528
Lys His Thr Ala Asn Ser His His Leu Ser Ala Leu Val Thr Pro Ala
                165                 170                 175

ggg atg tcc tat gag tgc cag gct cag cag acc att tct ctg gcc tcc 576
Gly Met Ser Tyr Glu Cys Gln Ala Gln Gln Thr Ile Ser Leu Ala Ser
            180                 185                 190

agt gat cct cag aag act gtc acc atg atc ctg tct gca gtg cac att 624
Ser Asp Pro Gln Lys Thr Val Thr Met Ile Leu Ser Ala Val His Ile
        195                 200                 205

cag ccc ttt gat atc atc tct gac ttt gtc ttc agt gaa gag cat aaa 672
Gln Pro Phe Asp Ile Ile Ser Asp Phe Val Phe Ser Glu Glu His Lys
    210                 215                 220

tgt cca gtg gat gag cga gaa cag ttg gaa gag acc ctg ccc ctg atc 720
Cys Pro Val Asp Glu Arg Glu Gln Leu Glu Glu Thr Leu Pro Leu Ile
225                 230                 235                 240

ctg ggc ctc atc tta ggc ctg gtc att gtc ata aca ctt gtg att tac 768
Leu Gly Leu Ile Leu Gly Leu Val Ile Val Ile Thr Leu Val Ile Tyr
                245                 250                 255

cac att cat cac aaa atg act gcc aac caa gtg cag atc ccc aga gac 816
His Ile His His Lys Met Thr Ala Asn Gln Val Gln Ile Pro Arg Asp
            260                 265                 270

cgt tcc cag tac aag cac atg ggc tag 843
Arg Ser Gln Tyr Lys His Met Gly
        275                 280

<210> 6
<211> 280
<212> PRT

<213>  Rattus norvegicus

<400>  6

Met Asp Leu Arg Gly Arg Ala Leu Leu Gly Gly Asp Arg Leu Arg Ile
1               5                   10                  15

Leu Leu Met Phe Phe His Ala Met Ala Gln Thr Val Ala Glu Gln Glu
            20                  25                  30

Val Glu Asn Leu Ser Gly Leu Ser Thr Asn Pro Glu Lys Asp Ile Phe
        35                  40                  45

Val Val Arg Glu Asn Gly Thr Thr Cys Leu Met Ala Glu Phe Ala Ala
    50                  55                  60

Lys Phe Ile Val Pro Tyr Asp Val Trp Ala Ser Asn Tyr Val Asp Leu
65                  70                  75                  80

Ile Thr Glu Gln Ala Glu Ile Ser Leu Thr Arg Gly Ala Glu Val Lys
                85                  90                  95

Gly Arg Cys Gly His Asn Glu Ser Glu Leu Gln Val Phe Trp Val Asp
            100                 105                 110

Arg Ala Tyr Thr Leu Lys Met Leu Phe Val Lys Glu Ser His Asn Thr
        115                 120                 125

Ser Lys Gly Leu Glu Ala Thr Trp Lys Leu Ser Lys Val Gln Phe Val
    130                 135                 140

Tyr Asp Ser Ser Glu Lys Thr His Phe Lys Asp Ala Val Ser Ala Gly
145                 150                 155                 160

Lys His Thr Ala Asn Ser His His Leu Ser Ala Leu Val Thr Pro Ala
                165                 170                 175

Gly Met Ser Tyr Glu Cys Gln Ala Gln Gln Thr Ile Ser Leu Ala Ser
            180                 185                 190

Ser Asp Pro Gln Lys Thr Val Thr Met Ile Leu Ser Ala Val His Ile
        195                 200                 205

Gln Pro Phe Asp Ile Ile Ser Asp Phe Val Phe Ser Glu Glu His Lys
    210                 215                 220

Cys Pro Val Asp Glu Arg Glu Gln Leu Glu Glu Thr Leu Pro Leu Ile

225                 230                 235                 240

Leu Gly Leu Ile Leu Gly Leu Val Ile Val Ile Thr Leu Val Ile Tyr
                245                 250                 255

His Ile His His Lys Met Thr Ala Asn Gln Val Gln Ile Pro Arg Asp
            260                 265                 270

Arg Ser Gln Tyr Lys His Met Gly
        275                 280

<210>  7
<211>  843
<212>  DNA
<213>  Bos taurus

<220>
<221>  CDS
<222>  (1)..(843)

<400>  7
atg gat ctc cgc aga aga gcc ctt ctc ggc gtg gat gga ctt cga gtt       48
Met Asp Leu Arg Arg Arg Ala Leu Leu Gly Val Asp Gly Leu Arg Val
1               5                   10                  15

ctc ctg atg ctg ttc cat aca gtg act cga atc atg gca gaa caa gaa       96
Leu Leu Met Leu Phe His Thr Val Thr Arg Ile Met Ala Glu Gln Glu
            20                  25                  30

gtg gaa aat ctc tca ggc ctt tcc act aac cct gaa aaa gat ata ttt      144
Val Glu Asn Leu Ser Gly Leu Ser Thr Asn Pro Glu Lys Asp Ile Phe
        35                  40                  45

gtg gtg cgg gaa aat ggg acg acg tgt ctc atg gca gag ttt gca gcc      192
Val Val Arg Glu Asn Gly Thr Thr Cys Leu Met Ala Glu Phe Ala Ala
    50                  55                  60

aaa ttt att gtc cct tat gat gtg tgg gcc agc aat tac gtg gat ctg      240
Lys Phe Ile Val Pro Tyr Asp Val Trp Ala Ser Asn Tyr Val Asp Leu
65                  70                  75                  80

atc acg gag caa gcg gat atc tca ttg acc cgg gga gcc gag gtg aag      288
Ile Thr Glu Gln Ala Asp Ile Ser Leu Thr Arg Gly Ala Glu Val Lys
                85                  90                  95

ggc cac tgt ggc cac gac gag tcg gag ctg cag gtg ttc tgg gtg gat      336
Gly His Cys Gly His Asp Glu Ser Glu Leu Gln Val Phe Trp Val Asp
            100                 105                 110

cgc gcg tac gca ctc aaa atg ctg ttt cta aag gaa agt cac aac acg      384
Arg Ala Tyr Ala Leu Lys Met Leu Phe Leu Lys Glu Ser His Asn Thr
        115                 120                 125

ccc aag gga ccc gag gca acg tgg aag ctg agc aaa gtc cag ttt gtc      432
Pro Lys Gly Pro Glu Ala Thr Trp Lys Leu Ser Lys Val Gln Phe Val
    130                 135                 140

tac gac tcc tca gag aag acc cac ttt aaa gac gca gtc agt gct ggg 480
Tyr Asp Ser Ser Glu Lys Thr His Phe Lys Asp Ala Val Ser Ala Gly
145                 150                 155                 160

aag cac aca gcc aac tcg cat cac ctc tct gcc ttg gtc acc cca gcc 528
Lys His Thr Ala Asn Ser His His Leu Ser Ala Leu Val Thr Pro Ala
                165                 170                 175

ggg aag tcc tat gaa tgt caa gct cag cag acc atc tca cta gcc tcc 576
Gly Lys Ser Tyr Glu Cys Gln Ala Gln Gln Thr Ile Ser Leu Ala Ser
            180                 185                 190

agc gac ccg cag aag aca gtc acc atg atc ctg tcc gcg gtc cac atc 624
Ser Asp Pro Gln Lys Thr Val Thr Met Ile Leu Ser Ala Val His Ile
        195                 200                 205

cag ccc ttt gac atc atc tct gac ttt gtc ttc agt gaa gag cat aaa 672
Gln Pro Phe Asp Ile Ile Ser Asp Phe Val Phe Ser Glu Glu His Lys
    210                 215                 220

tgc cca gtg gat gag cgg gag cag ctg gag gaa acc ttg ccg ctg att 720
Cys Pro Val Asp Glu Arg Glu Gln Leu Glu Glu Thr Leu Pro Leu Ile
225                 230                 235                 240

ttg ggg ctc atc ttg ggc ctc gtc att gtg gtc aca ctg gtg att tac 768
Leu Gly Leu Ile Leu Gly Leu Val Ile Val Val Thr Leu Val Ile Tyr
                245                 250                 255

cac atc cac cac aag atg act gcc aac cag gtc cag atc cct agg gac 816
His Ile His His Lys Met Thr Ala Asn Gln Val Gln Ile Pro Arg Asp
            260                 265                 270

cgg tcc cag tat aag cac atg ggc tag 843
Arg Ser Gln Tyr Lys His Met Gly
        275                 280

<210> 8
<211> 280
<212> PRT
<213> Bos taurus

<400> 8

Met Asp Leu Arg Arg Arg Ala Leu Leu Gly Val Asp Gly Leu Arg Val
1               5                   10                  15

Leu Leu Met Leu Phe His Thr Val Thr Arg Ile Met Ala Glu Gln Glu
            20                  25                  30

Val Glu Asn Leu Ser Gly Leu Ser Thr Asn Pro Glu Lys Asp Ile Phe
        35                  40                  45

Val Val Arg Glu Asn Gly Thr Thr Cys Leu Met Ala Glu Phe Ala Ala
    50                  55                  60

Lys Phe Ile Val Pro Tyr Asp Val Trp Ala Ser Asn Tyr Val Asp Leu
65                  70                  75                  80

Ile Thr Glu Gln Ala Asp Ile Ser Leu Thr Arg Gly Ala Glu Val Lys
                85                  90                  95

Gly His Cys Gly His Asp Glu Ser Glu Leu Gln Val Phe Trp Val Asp
            100                 105                 110

Arg Ala Tyr Ala Leu Lys Met Leu Phe Leu Lys Glu Ser His Asn Thr
        115                 120                 125

Pro Lys Gly Pro Glu Ala Thr Trp Lys Leu Ser Lys Val Gln Phe Val
    130                 135                 140

Tyr Asp Ser Ser Glu Lys Thr His Phe Lys Asp Ala Val Ser Ala Gly
145                 150                 155                 160

Lys His Thr Ala Asn Ser His His Leu Ser Ala Leu Val Thr Pro Ala
                165                 170                 175

Gly Lys Ser Tyr Glu Cys Gln Ala Gln Gln Thr Ile Ser Leu Ala Ser
            180                 185                 190

Ser Asp Pro Gln Lys Thr Val Thr Met Ile Leu Ser Ala Val His Ile
        195                 200                 205

Gln Pro Phe Asp Ile Ile Ser Asp Phe Val Phe Ser Glu Glu His Lys
    210                 215                 220

Cys Pro Val Asp Glu Arg Glu Gln Leu Glu Glu Thr Leu Pro Leu Ile
225                 230                 235                 240

Leu Gly Leu Ile Leu Gly Leu Val Ile Val Val Thr Leu Val Ile Tyr
                245                 250                 255

His Ile His His Lys Met Thr Ala Asn Gln Val Gln Ile Pro Arg Asp
            260                 265                 270

Arg Ser Gln Tyr Lys His Met Gly
        275                 280

<210>  9
<211>  843
<212>  DNA
<213>  Pan troglodytes

<220>
<221> CDS
<222> (1)..(843)

<400> 9
atg gat ctc cga gga aga gcg gtc ccc agc atc gac aga ctt cga gtt 48
Met Asp Leu Arg Gly Arg Ala Val Pro Ser Ile Asp Arg Leu Arg Val
1               5                   10                  15

ctc ctg atg ttg ttc cat aca atg gct caa atc atg gca gaa caa gaa 96
Leu Leu Met Leu Phe His Thr Met Ala Gln Ile Met Ala Glu Gln Glu
            20                  25                  30

gtg gaa aat ctc tca ggc ctt tcc act aac cct gaa aaa gat ata ttt 144
Val Glu Asn Leu Ser Gly Leu Ser Thr Asn Pro Glu Lys Asp Ile Phe
        35                  40                  45

gtg gtg cgg gaa aat ggg acg acg tgt ctc atg gca gag ttt gca gcc 192
Val Val Arg Glu Asn Gly Thr Thr Cys Leu Met Ala Glu Phe Ala Ala
    50                  55                  60

aaa ttt att gta cct tat gat gtg tgg gcc agc aat tac gta gat ctg 240
Lys Phe Ile Val Pro Tyr Asp Val Trp Ala Ser Asn Tyr Val Asp Leu
65                  70                  75                  80

atc aca gaa cag gcc gat atc gca ttg acc cgg gga gct gag gtg aag 288
Ile Thr Glu Gln Ala Asp Ile Ala Leu Thr Arg Gly Ala Glu Val Lys
                85                  90                  95

ggc cgc tgt ggc cac agc gag tcg gag ctg caa gtg ttc tgg gtg gat 336
Gly Arg Cys Gly His Ser Glu Ser Glu Leu Gln Val Phe Trp Val Asp
            100                 105                 110

cgc gca tat gca ctc aaa atg ctc ttt gta aag gaa agc cac aac atg 384
Arg Ala Tyr Ala Leu Lys Met Leu Phe Val Lys Glu Ser His Asn Met
        115                 120                 125

tcc aag gga cct gag gcg act tgg agg ctg agc aaa gtg cag ttt gtc 432
Ser Lys Gly Pro Glu Ala Thr Trp Arg Leu Ser Lys Val Gln Phe Val
    130                 135                 140

tac gac tcc tcg gag aaa acc cac ttc aaa gac gca gtc agt gct ggg 480
Tyr Asp Ser Ser Glu Lys Thr His Phe Lys Asp Ala Val Ser Ala Gly
145                 150                 155                 160

aag cac aca gcc aac tcg cac cac ctc tct gcc ttg gtc acc ccc gct 528
Lys His Thr Ala Asn Ser His His Leu Ser Ala Leu Val Thr Pro Ala
                165                 170                 175

ggg aag tcc tat gag tgt caa gct caa caa acc att tca ctg gcc tct 576
Gly Lys Ser Tyr Glu Cys Gln Ala Gln Gln Thr Ile Ser Leu Ala Ser
            180                 185                 190

agt gat ctg cag aag acg gtc acc atg atc ctg tct gcg gtc cac atc 624
Ser Asp Leu Gln Lys Thr Val Thr Met Ile Leu Ser Ala Val His Ile
        195                 200                 205

caa cct ttt gac att atc tca gat ttt gtc ttc agt gaa gag cat aaa 672
Gln Pro Phe Asp Ile Ile Ser Asp Phe Val Phe Ser Glu Glu His Lys
    210                 215                 220

tgc cca gtg gat gag cgg gag caa ctg gaa gaa acc ttg ccc ctg att 720
Cys Pro Val Asp Glu Arg Glu Gln Leu Glu Glu Thr Leu Pro Leu Ile
225                 230                 235                 240

ttg ggg ctc atc ttg ggc ctc gtc atc atg gta aca ctc gcg att tac 768
Leu Gly Leu Ile Leu Gly Leu Val Ile Met Val Thr Leu Ala Ile Tyr
                245                 250                 255

cac gtc cac cac aaa atg act gcc aac cag gtg cag atc cct cgg gac 816
His Val His His Lys Met Thr Ala Asn Gln Val Gln Ile Pro Arg Asp
            260                 265                 270

aga tcc cag tat aag cac atg ggc tag 843
Arg Ser Gln Tyr Lys His Met Gly
        275                 280

<210> 10
<211> 280
<212> PRT
<213> Pan troglodytes

<400> 10

Met Asp Leu Arg Gly Arg Ala Val Pro Ser Ile Asp Arg Leu Arg Val
1               5                   10                  15

Leu Leu Met Leu Phe His Thr Met Ala Gln Ile Met Ala Glu Gln Glu
            20                  25                  30

Val Glu Asn Leu Ser Gly Leu Ser Thr Asn Pro Glu Lys Asp Ile Phe
        35                  40                  45

Val Val Arg Glu Asn Gly Thr Thr Cys Leu Met Ala Glu Phe Ala Ala
    50                  55                  60

Lys Phe Ile Val Pro Tyr Asp Val Trp Ala Ser Asn Tyr Val Asp Leu
65                  70                  75                  80

Ile Thr Glu Gln Ala Asp Ile Ala Leu Thr Arg Gly Ala Glu Val Lys
                85                  90                  95

Gly Arg Cys Gly His Ser Glu Ser Glu Leu Gln Val Phe Trp Val Asp
            100                 105                 110

Arg Ala Tyr Ala Leu Lys Met Leu Phe Val Lys Glu Ser His Asn Met
        115                 120                 125

Ser Lys Gly Pro Glu Ala Thr Trp Arg Leu Ser Lys Val Gln Phe Val
    130                 135                 140

Tyr Asp Ser Ser Glu Lys Thr His Phe Lys Asp Ala Val Ser Ala Gly
145                 150                 155                 160

Lys His Thr Ala Asn Ser His His Leu Ser Ala Leu Val Thr Pro Ala
                165                 170                 175

Gly Lys Ser Tyr Glu Cys Gln Ala Gln Gln Thr Ile Ser Leu Ala Ser
            180                 185                 190

Ser Asp Leu Gln Lys Thr Val Thr Met Ile Leu Ser Ala Val His Ile
        195                 200                 205

Gln Pro Phe Asp Ile Ile Ser Asp Phe Val Phe Ser Glu Glu His Lys
    210                 215                 220

Cys Pro Val Asp Glu Arg Glu Gln Leu Glu Glu Thr Leu Pro Leu Ile
225                 230                 235                 240

Leu Gly Leu Ile Leu Gly Leu Val Ile Met Val Thr Leu Ala Ile Tyr
                245                 250                 255

His Val His His Lys Met Thr Ala Asn Gln Val Gln Ile Pro Arg Asp
            260                 265                 270

Arg Ser Gln Tyr Lys His Met Gly
        275                 280

<210>  11
<211>  843
<212>  DNA
<213>  Monodelphis domestica

<220>
<221>  CDS
<222>  (1)..(843)

<400>  11
atg gac cac agg agg aga act gct ccc gga atc ttg agt ctt caa gtc       48
Met Asp His Arg Arg Arg Thr Ala Pro Gly Ile Leu Ser Leu Gln Val
1               5                   10                  15

cta ttc ttg ctt ttc cat aca att gct cag atc atg gca gaa caa gaa       96
Leu Phe Leu Leu Phe His Thr Ile Ala Gln Ile Met Ala Glu Gln Glu
            20                  25                  30

gtt gaa aat ctc tca ggt ctt tcc act aac cct gaa aaa gat ata ttt      144
Val Glu Asn Leu Ser Gly Leu Ser Thr Asn Pro Glu Lys Asp Ile Phe
        35                  40                  45

gtg gtg cgg gaa aat ggg aca acg tgt cta atg gca gaa ttt gca gca      192
Val Val Arg Glu Asn Gly Thr Thr Cys Leu Met Ala Glu Phe Ala Ala

    50                  55                  60

aaa ttt att gta cct tat gat gta tgg gcc agc aat tat gtc gat ctg 240
Lys Phe Ile Val Pro Tyr Asp Val Trp Ala Ser Asn Tyr Val Asp Leu
65                  70                  75                  80

atc acg gaa caa gct gat att cca ctt tcc cgg ggg gct gaa atg aag 288
Ile Thr Glu Gln Ala Asp Ile Pro Leu Ser Arg Gly Ala Glu Met Lys
                85                  90                  95

ggc cgg tgt gga cac aat gaa tcc gag ttg caa gtc ttc tgg gtc gac 336
Gly Arg Cys Gly His Asn Glu Ser Glu Leu Gln Val Phe Trp Val Asp
            100                 105                 110

aaa gct tat gcc ctc aaa atg ctc ttt gtg aag gaa agc cac aac aca 384
Lys Ala Tyr Ala Leu Lys Met Leu Phe Val Lys Glu Ser His Asn Thr
        115                 120                 125

tca aag ggc cta gaa gct tcc tgg agg ctg agt aaa gtt caa ttc gtt 432
Ser Lys Gly Leu Glu Ala Ser Trp Arg Leu Ser Lys Val Gln Phe Val
    130                 135                 140

tat gat tcc tct gag aaa acc tac ttt aaa gac gcg gtc aat cct ggg 480
Tyr Asp Ser Ser Glu Lys Thr Tyr Phe Lys Asp Ala Val Asn Pro Gly
145                 150                 155                 160

aag cat aca gcc aat tct cat cgc cta tct gcc tta gtc acc cca gcc 528
Lys His Thr Ala Asn Ser His Arg Leu Ser Ala Leu Val Thr Pro Ala
                165                 170                 175

ggg aaa tcc tat gaa tgc caa gct caa cag acc atc tct ctc acc tcc 576
Gly Lys Ser Tyr Glu Cys Gln Ala Gln Gln Thr Ile Ser Leu Thr Ser
            180                 185                 190

agt gat cat cag aaa aca gtt gct atg ctc ctc tca tca gtc cac atc 624
Ser Asp His Gln Lys Thr Val Ala Met Leu Leu Ser Ser Val His Ile
        195                 200                 205

cag ccc ttt gac atc atc tct gac ttt gtt ttc agt gaa gaa cac aag 672
Gln Pro Phe Asp Ile Ile Ser Asp Phe Val Phe Ser Glu Glu His Lys
    210                 215                 220

tgc cct gtg gac gag agg gaa caa ctg gaa gaa act ttg cct ctg att 720
Cys Pro Val Asp Glu Arg Glu Gln Leu Glu Glu Thr Leu Pro Leu Ile
225                 230                 235                 240

ttg ggg ctg atc ttg gga ctg gtc att gtg ata aca ctg aca atc tac 768
Leu Gly Leu Ile Leu Gly Leu Val Ile Val Ile Thr Leu Thr Ile Tyr
                245                 250                 255

cac atc cat cac aaa ctg aca gcc aat cag gtg cag att cca agg gac 816
His Ile His His Lys Leu Thr Ala Asn Gln Val Gln Ile Pro Arg Asp
            260                 265                 270

aga tct cag tac aaa cac atg ggc tag 843
Arg Ser Gln Tyr Lys His Met Gly
        275                 280

<210> 12
<211> 280

<212>  PRT
<213>  Monodelphis domestica

<400>  12

Met Asp His Arg Arg Arg Thr Ala Pro Gly Ile Leu Ser Leu Gln Val
1               5                   10                  15

Leu Phe Leu Leu Phe His Thr Ile Ala Gln Ile Met Ala Glu Gln Glu
            20                  25                  30

Val Glu Asn Leu Ser Gly Leu Ser Thr Asn Pro Glu Lys Asp Ile Phe
        35                  40                  45

Val Val Arg Glu Asn Gly Thr Thr Cys Leu Met Ala Glu Phe Ala Ala
    50                  55                  60

Lys Phe Ile Val Pro Tyr Asp Val Trp Ala Ser Asn Tyr Val Asp Leu
65                  70                  75                  80

Ile Thr Glu Gln Ala Asp Ile Pro Leu Ser Arg Gly Ala Glu Met Lys
                85                  90                  95

Gly Arg Cys Gly His Asn Glu Ser Glu Leu Gln Val Phe Trp Val Asp
            100                 105                 110

Lys Ala Tyr Ala Leu Lys Met Leu Phe Val Lys Glu Ser His Asn Thr
        115                 120                 125

Ser Lys Gly Leu Glu Ala Ser Trp Arg Leu Ser Lys Val Gln Phe Val
    130                 135                 140

Tyr Asp Ser Ser Glu Lys Thr Tyr Phe Lys Asp Ala Val Asn Pro Gly
145                 150                 155                 160

Lys His Thr Ala Asn Ser His Arg Leu Ser Ala Leu Val Thr Pro Ala
                165                 170                 175

Gly Lys Ser Tyr Glu Cys Gln Ala Gln Gln Thr Ile Ser Leu Thr Ser
            180                 185                 190

Ser Asp His Gln Lys Thr Val Ala Met Leu Leu Ser Ser Val His Ile
        195                 200                 205

Gln Pro Phe Asp Ile Ile Ser Asp Phe Val Phe Ser Glu Glu His Lys
    210                 215                 220

Cys Pro Val Asp Glu Arg Glu Gln Leu Glu Glu Thr Leu Pro Leu Ile
225                 230                 235                 240

Leu Gly Leu Ile Leu Gly Leu Val Ile Val Ile Thr Leu Thr Ile Tyr
                245                 250                 255

His Ile His His Lys Leu Thr Ala Asn Gln Val Gln Ile Pro Arg Asp
            260                 265                 270

Arg Ser Gln Tyr Lys His Met Gly
        275                 280

<210>  13
<211>  756
<212>  DNA
<213>  Xenopus tropicalis

<220>
<221>  CDS
<222>  (1)..(756)

<400>  13
ctt gcg gag caa gaa gtc gaa aac ctc tca ggt ctc tcc agt aac cca      48
Leu Ala Glu Gln Glu Val Glu Asn Leu Ser Gly Leu Ser Ser Asn Pro
1               5                   10                  15

gac aaa aat att ttt gct atc cgg gag aat ggg acg act tgc ctt atg      96
Asp Lys Asn Ile Phe Ala Ile Arg Glu Asn Gly Thr Thr Cys Leu Met
            20                  25                  30

gct gaa ttc tca gct aga atc ctc gta ccc tat gaa gta cct tcc agt      144
Ala Glu Phe Ser Ala Arg Ile Leu Val Pro Tyr Glu Val Pro Ser Ser
        35                  40                  45

aat gaa gtg gat tgg gac cta gaa gaa gcc tct atc cag tta ccc aga      192
Asn Glu Val Asp Trp Asp Leu Glu Glu Ala Ser Ile Gln Leu Pro Arg
    50                  55                  60

gat aca gaa ata aga ggg aaa tgt tgg aac aat gaa tct gag ctc cat      240
Asp Thr Glu Ile Arg Gly Lys Cys Trp Asn Asn Glu Ser Glu Leu His
65                  70                  75                  80

ctg tcc tgg ctg gac aaa gcc tat act ctc aag ctc ttt ttc tcc aag      288
Leu Ser Trp Leu Asp Lys Ala Tyr Thr Leu Lys Leu Phe Phe Ser Lys
                85                  90                  95

gag gga caa gat gct tcc aaa tcc cgt agt tgg aaa atg agt aaa atc      336
Glu Gly Gln Asp Ala Ser Lys Ser Arg Ser Trp Lys Met Ser Lys Ile
            100                 105                 110

cag ttt ttg tat gac cct tcg gaa cac aca att ttt aag agc gga gca      384
Gln Phe Leu Tyr Asp Pro Ser Glu His Thr Ile Phe Lys Ser Gly Ala
        115                 120                 125

aga cct gga agg cac aca gcc aat agc cac cac ctc tct ctc atg gtc      432
Arg Pro Gly Arg His Thr Ala Asn Ser His His Leu Ser Leu Met Val

130                 135                 140

aca cca gct ggc atg tct tat gaa tgt gag gca acc cag aga atc agc       480
Thr Pro Ala Gly Met Ser Tyr Glu Cys Glu Ala Thr Gln Arg Ile Ser
145                 150                 155                 160

ctg aca tct act gac cac cag aag ata gtc gtt ctg tac ctt tcg gaa       528
Leu Thr Ser Thr Asp His Gln Lys Ile Val Val Leu Tyr Leu Ser Glu
                165                 170                 175

gtt cac cta cag cca ttt gat atc aag agt gat ttc gtc tac agt gaa       576
Val His Leu Gln Pro Phe Asp Ile Lys Ser Asp Phe Val Tyr Ser Glu
            180                 185                 190

gaa tac aag tgt cca acg gat caa agg aag cag ctg gag gaa acg ctt       624
Glu Tyr Lys Cys Pro Thr Asp Gln Arg Lys Gln Leu Glu Glu Thr Leu
        195                 200                 205

ccc ctc atc cta ggc ctg acg ttg gga gta gcc ata ctc atc atc gtc       672
Pro Leu Ile Leu Gly Leu Thr Leu Gly Val Ala Ile Leu Ile Ile Val
    210                 215                 220

gcc gtg tat cac att cac cac aag atg aca gcc aat caa gtt cag ata       720
Ala Val Tyr His Ile His His Lys Met Thr Ala Asn Gln Val Gln Ile
225                 230                 235                 240

cct cgg gac aga tct ctg tac aag cac atg gga taa                       756
Pro Arg Asp Arg Ser Leu Tyr Lys His Met Gly
                245                 250


<210>  14
<211>  251
<212>  PRT
<213>  Xenopus tropicalis

<400>  14

Leu Ala Glu Gln Glu Val Glu Asn Leu Ser Gly Leu Ser Ser Asn Pro
1               5                   10                  15


Asp Lys Asn Ile Phe Ala Ile Arg Glu Asn Gly Thr Thr Cys Leu Met
            20                  25                  30


Ala Glu Phe Ser Ala Arg Ile Leu Val Pro Tyr Glu Val Pro Ser Ser
        35                  40                  45


Asn Glu Val Asp Trp Asp Leu Glu Glu Ala Ser Ile Gln Leu Pro Arg
    50                  55                  60


Asp Thr Glu Ile Arg Gly Lys Cys Trp Asn Asn Glu Ser Glu Leu His
65                  70                  75                  80


Leu Ser Trp Leu Asp Lys Ala Tyr Thr Leu Lys Leu Phe Phe Ser Lys
                85                  90                  95

Glu Gly Gln Asp Ala Ser Lys Ser Arg Ser Trp Lys Met Ser Lys Ile
            100                 105                 110

Gln Phe Leu Tyr Asp Pro Ser Glu His Thr Ile Phe Lys Ser Gly Ala
        115                 120                 125

Arg Pro Gly Arg His Thr Ala Asn Ser His His Leu Ser Leu Met Val
    130                 135                 140

Thr Pro Ala Gly Met Ser Tyr Glu Cys Glu Ala Thr Gln Arg Ile Ser
145                 150                 155                 160

Leu Thr Ser Thr Asp His Gln Lys Ile Val Val Leu Tyr Leu Ser Glu
                165                 170                 175

Val His Leu Gln Pro Phe Asp Ile Lys Ser Asp Phe Val Tyr Ser Glu
            180                 185                 190

Glu Tyr Lys Cys Pro Thr Asp Gln Arg Lys Gln Leu Glu Glu Thr Leu
        195                 200                 205

Pro Leu Ile Leu Gly Leu Thr Leu Gly Val Ala Ile Leu Ile Ile Val
    210                 215                 220

Ala Val Tyr His Ile His His Lys Met Thr Ala Asn Gln Val Gln Ile
225                 230                 235                 240

Pro Arg Asp Arg Ser Leu Tyr Lys His Met Gly
                245                 250

<210> 15
<211> 846
<212> DNA
<213> Gallus gallus

<220>
<221> CDS
<222> (1)..(846)

<400> 15
gcg gac acg tcg ggc cga aca ggg agg atg gcc gcg ggg cgc ctc ccg 48
Ala Asp Thr Ser Gly Arg Thr Gly Arg Met Ala Ala Gly Arg Leu Pro
1               5                   10                  15

ggg ctg ctc ttc ctc ttg cac gcc gca gct cgt ctg gct gcc gag caa 96
Gly Leu Leu Phe Leu Leu His Ala Ala Ala Arg Leu Ala Ala Glu Gln
            20                  25                  30

gaa gtt gaa aac ctc tcc ggg ctc tcc cct aac ccc gaa aag gat atc 144

Glu Val Glu Asn Leu Ser Gly Leu Ser Pro Asn Pro Glu Lys Asp Ile
        35                  40                  45

ttt gtg gtg cgg gag aac agg acg acg tgc ctc atg gcg gaa ttc gcc 192
Phe Val Val Arg Glu Asn Arg Thr Thr Cys Leu Met Ala Glu Phe Ala
    50                  55                  60

gcc aag ttc gtc gtc cct tac gac gtg tgg gcc agc aac tac gtg gat 240
Ala Lys Phe Val Val Pro Tyr Asp Val Trp Ala Ser Asn Tyr Val Asp
65                  70                  75                  80

ctg atc acg gag caa gcc gac att ccg ctg tcg cgg ggc gcc gaa atg 288
Leu Ile Thr Glu Gln Ala Asp Ile Pro Leu Ser Arg Gly Ala Glu Met
                85                  90                  95

aag ggc aag tgc ggc acc aac gag tcg gag ctg gag atc tcc tgg ctg 336
Lys Gly Lys Cys Gly Thr Asn Glu Ser Glu Leu Glu Ile Ser Trp Leu
            100                 105                 110

gag cgg gcg tac acc ctc aaa ctc ttc ttc ctg aag gag ggg cac aac 384
Glu Arg Ala Tyr Thr Leu Lys Leu Phe Phe Leu Lys Glu Gly His Asn
        115                 120                 125

acg tcc cgc ggg ccg gag gct ttc tgg agg ctc agc cgg atc cag ttc 432
Thr Ser Arg Gly Pro Glu Ala Phe Trp Arg Leu Ser Arg Ile Gln Phe
    130                 135                 140

tcc tac gac acc tcc gag cgc acc tac ttc aag gac gcc gtc agc cct 480
Ser Tyr Asp Thr Ser Glu Arg Thr Tyr Phe Lys Asp Ala Val Ser Pro
145                 150                 155                 160

ggg aag cat aca gcc agc tcc cat cgg ctc tct gcc ttg gtt acc cca 528
Gly Lys His Thr Ala Ser Ser His Arg Leu Ser Ala Leu Val Thr Pro
                165                 170                 175

gcg ggg aag tcc tac gag tgc cag gca cag cag acc atc tcc ctc atc 576
Ala Gly Lys Ser Tyr Glu Cys Gln Ala Gln Gln Thr Ile Ser Leu Ile
            180                 185                 190

tcc agt gac cat cag aag tct gtg cag ctc ttg ttg tct gaa gtc cgc 624
Ser Ser Asp His Gln Lys Ser Val Gln Leu Leu Leu Ser Glu Val Arg
        195                 200                 205

atc cag ccc ttt gac atc act gca gat ttt gtc ttc agt gaa gaa cac 672
Ile Gln Pro Phe Asp Ile Thr Ala Asp Phe Val Phe Ser Glu Glu His
    210                 215                 220

aag tgc ccg gtg gac cag agg gag cag ttg gaa gaa act ctg cct ctg 720
Lys Cys Pro Val Asp Gln Arg Glu Gln Leu Glu Glu Thr Leu Pro Leu
225                 230                 235                 240

att ttg ggc ctg ata ctg ggg ttg gtt atc gtg ata acc ctc tgt gtt 768
Ile Leu Gly Leu Ile Leu Gly Leu Val Ile Val Ile Thr Leu Cys Val
                245                 250                 255

tac cac atc cac cac aag ctc aca gcc aac caa gtg caa att cct cgt 816
Tyr His Ile His His Lys Leu Thr Ala Asn Gln Val Gln Ile Pro Arg
            260                 265                 270

gac aga tct cag tat aaa cac atg gga tag 846
Asp Arg Ser Gln Tyr Lys His Met Gly

275                 280

<210>  16
<211>  281
<212>  PRT
<213>  Gallus gallus

<400>  16

Ala Asp Thr Ser Gly Arg Thr Gly Arg Met Ala Ala Gly Arg Leu Pro
1               5                   10                  15

Gly Leu Leu Phe Leu Leu His Ala Ala Ala Arg Leu Ala Ala Glu Gln
            20                  25                  30

Glu Val Glu Asn Leu Ser Gly Leu Ser Pro Asn Pro Glu Lys Asp Ile
        35                  40                  45

Phe Val Val Arg Glu Asn Arg Thr Thr Cys Leu Met Ala Glu Phe Ala
    50                  55                  60

Ala Lys Phe Val Val Pro Tyr Asp Val Trp Ala Ser Asn Tyr Val Asp
65                  70                  75                  80

Leu Ile Thr Glu Gln Ala Asp Ile Pro Leu Ser Arg Gly Ala Glu Met
                85                  90                  95

Lys Gly Lys Cys Gly Thr Asn Glu Ser Glu Leu Glu Ile Ser Trp Leu
            100                 105                 110

Glu Arg Ala Tyr Thr Leu Lys Leu Phe Phe Leu Lys Glu Gly His Asn
        115                 120                 125

Thr Ser Arg Gly Pro Glu Ala Phe Trp Arg Leu Ser Arg Ile Gln Phe
    130                 135                 140

Ser Tyr Asp Thr Ser Glu Arg Thr Tyr Phe Lys Asp Ala Val Ser Pro
145                 150                 155                 160

Gly Lys His Thr Ala Ser Ser His Arg Leu Ser Ala Leu Val Thr Pro
                165                 170                 175

Ala Gly Lys Ser Tyr Glu Cys Gln Ala Gln Gln Thr Ile Ser Leu Ile
            180                 185                 190

Ser Ser Asp His Gln Lys Ser Val Gln Leu Leu Leu Ser Glu Val Arg
        195                 200                 205

Ile Gln Pro Phe Asp Ile Thr Ala Asp Phe Val Phe Ser Glu Glu His
    210                 215                 220

Lys Cys Pro Val Asp Gln Arg Glu Gln Leu Glu Glu Thr Leu Pro Leu
225                 230                 235                 240

Ile Leu Gly Leu Ile Leu Gly Leu Val Ile Val Ile Thr Leu Cys Val
                245                 250                 255

Tyr His Ile His His Lys Leu Thr Ala Asn Gln Val Gln Ile Pro Arg
            260                 265                 270

Asp Arg Ser Gln Tyr Lys His Met Gly
        275                 280

<210>  17
<211>  801
<212>  DNA
<213>  Anopheles gambiae

<220>
<221>  CDS
<222>  (1)..(801)

<400>  17
att ttc gtt cat ctt cta gcc aca ctg ctg cac ctc tgc aac act ctg       48
Ile Phe Val His Leu Leu Ala Thr Leu Leu His Leu Cys Asn Thr Leu
1               5                   10                  15

cag ctg gaa gga ctc tcg acc aag aag cca cgc att aca aag tac acg       96
Gln Leu Glu Gly Leu Ser Thr Lys Lys Pro Arg Ile Thr Lys Tyr Thr
            20                  25                  30

atg aag ccc acg tcc agc tcg gta ccg gtg cca tcc tcc ctg tcg ctg      144
Met Lys Pro Thr Ser Ser Ser Val Pro Val Pro Ser Ser Leu Ser Leu
        35                  40                  45

tac cgc ctg gac gga tcg agt gga gca acg tgc ata ctg att caa acc      192
Tyr Arg Leu Asp Gly Ser Ser Gly Ala Thr Cys Ile Leu Ile Gln Thr
    50                  55                  60

gat gcc ctg cta agc att cag tac cgc gat aag tac aat gag gat aag      240
Asp Ala Leu Leu Ser Ile Gln Tyr Arg Asp Lys Tyr Asn Glu Asp Lys
65                  70                  75                  80

gaa gcg gac tcc ttc ctg ccg gac cag atg gac att tcc ggc gag tgc      288
Glu Ala Asp Ser Phe Leu Pro Asp Gln Met Asp Ile Ser Gly Glu Cys
                85                  90                  95

tgg gag gac gaa tcg cgc atc acc atg tcc tgg aag ggt ttc acc atc      336
Trp Glu Asp Glu Ser Arg Ile Thr Met Ser Trp Lys Gly Phe Thr Ile
            100                 105                 110

aac atc tac ttc tcg aag acg ccg ggc ggc gag cgc tgg tac gtg aac      384

Asn Ile Tyr Phe Ser Lys Thr Pro Gly Gly Glu Arg Trp Tyr Val Asn
        115                 120                 125

agc gtc gac ctg gcg tac tcg tcc tcc aac aag ctg ttc gag cac atc        432
Ser Val Asp Leu Ala Tyr Ser Ser Ser Asn Lys Leu Phe Glu His Ile
    130                 135                 140

gac cgt ccc ggg ctg gac gtg aag ctg tcg aca ccg ccc ggc acg ctg        480
Asp Arg Pro Gly Leu Asp Val Lys Leu Ser Thr Pro Pro Gly Thr Leu
145                 150                 155                 160

ctg ttc ccg acg ccg gtc ggc aag tcg tac acg tgc gac aac gag gtc        528
Leu Phe Pro Thr Pro Val Gly Lys Ser Tyr Thr Cys Asp Asn Glu Val
                165                 170                 175

atc atc acg atg ttc gcc cag gac gag aac gac aag tcg ggc cat ctg        576
Ile Ile Thr Met Phe Ala Gln Asp Glu Asn Asp Lys Ser Gly His Leu
            180                 185                 190

gcc aag ctg tat ctg cgc gag ctg cgg atg caa agc ttc atg tac aag        624
Ala Lys Leu Tyr Leu Arg Glu Leu Arg Met Gln Ser Phe Met Tyr Lys
        195                 200                 205

gcg ggc aat gcc tgg ggt ccc acg ttc cag tgc agc gca acc ggt acg        672
Ala Gly Asn Ala Trp Gly Pro Thr Phe Gln Cys Ser Ala Thr Gly Thr
    210                 215                 220

tac cgt gac gag acg gcc ccg atc gcc gtc ggt tcg acg ctg gcc gtg        720
Tyr Arg Asp Glu Thr Ala Pro Ile Ala Val Gly Ser Thr Leu Ala Val
225                 230                 235                 240

gcc acg gtc tgc acc gtt gtc gga tac gga ttg tgg aga tac ttc aaa        768
Ala Thr Val Cys Thr Val Val Gly Tyr Gly Leu Trp Arg Tyr Phe Lys
                245                 250                 255

gtc aag aag ttc cag tac gga act atg gct taa                            801
Val Lys Lys Phe Gln Tyr Gly Thr Met Ala
            260                 265

<210>  18
<211>  266
<212>  PRT
<213>  Anopheles gambiae

<400>  18

Ile Phe Val His Leu Leu Ala Thr Leu Leu His Leu Cys Asn Thr Leu
1               5                   10                  15

Gln Leu Glu Gly Leu Ser Thr Lys Lys Pro Arg Ile Thr Lys Tyr Thr
            20                  25                  30

Met Lys Pro Thr Ser Ser Ser Val Pro Val Pro Ser Ser Leu Ser Leu
        35                  40                  45

Tyr Arg Leu Asp Gly Ser Ser Gly Ala Thr Cys Ile Leu Ile Gln Thr
    50                  55                  60

Asp Ala Leu Leu Ser Ile Gln Tyr Arg Asp Lys Tyr Asn Glu Asp Lys
65                  70                  75                  80

Glu Ala Asp Ser Phe Leu Pro Asp Gln Met Asp Ile Ser Gly Glu Cys
                85                  90                  95

Trp Glu Asp Glu Ser Arg Ile Thr Met Ser Trp Lys Gly Phe Thr Ile
            100                 105                 110

Asn Ile Tyr Phe Ser Lys Thr Pro Gly Gly Glu Arg Trp Tyr Val Asn
        115                 120                 125

Ser Val Asp Leu Ala Tyr Ser Ser Ser Asn Lys Leu Phe Glu His Ile
    130                 135                 140

Asp Arg Pro Gly Leu Asp Val Lys Leu Ser Thr Pro Pro Gly Thr Leu
145                 150                 155                 160

Leu Phe Pro Thr Pro Val Gly Lys Ser Tyr Thr Cys Asp Asn Glu Val
                165                 170                 175

Ile Ile Thr Met Phe Ala Gln Asp Glu Asn Asp Lys Ser Gly His Leu
            180                 185                 190

Ala Lys Leu Tyr Leu Arg Glu Leu Arg Met Gln Ser Phe Met Tyr Lys
        195                 200                 205

Ala Gly Asn Ala Trp Gly Pro Thr Phe Gln Cys Ser Ala Thr Gly Thr
    210                 215                 220

Tyr Arg Asp Glu Thr Ala Pro Ile Ala Val Gly Ser Thr Leu Ala Val
225                 230                 235                 240

Ala Thr Val Cys Thr Val Val Gly Tyr Gly Leu Trp Arg Tyr Phe Lys
                245                 250                 255

Val Lys Lys Phe Gln Tyr Gly Thr Met Ala
            260                 265

<210>  19
<211>  870
<212>  DNA
<213>  Tetraodon nigroviridis

<220>

<221>  CDS
<222>  (1)..(870)

<400>  19
atg ggg cgg ctt gct tta ggc acc acg gag agc acc aaa tgt ttg ccg      48
Met Gly Arg Leu Ala Leu Gly Thr Thr Glu Ser Thr Lys Cys Leu Pro
1               5                   10                  15

ctt gcc gtg ttt ggt gtt ctg gcg ttg tct ctg gtg ctg gcg gag cag      96
Leu Ala Val Phe Gly Val Leu Ala Leu Ser Leu Val Leu Ala Glu Gln
            20                  25                  30

gaa ggc gag aac ctg tcc gga ctg tcc aac aac cca gac aaa gcc atc      144
Glu Gly Glu Asn Leu Ser Gly Leu Ser Asn Asn Pro Asp Lys Ala Ile
        35                  40                  45

ttt gcg gtt cgg gag aac gga acc aca tgt ctg atg gtg gag ttc gcc      192
Phe Ala Val Arg Glu Asn Gly Thr Thr Cys Leu Met Val Glu Phe Ala
    50                  55                  60

gta aaa ttc ctc gtg ccg tat gac gtg ctt gct ctc aat ggg ata gac      240
Val Lys Phe Leu Val Pro Tyr Asp Val Leu Ala Leu Asn Gly Ile Asp
65                  70                  75                  80

ctt att aca gaa cag gcg tcg ttt aca ctg cct cga agt gca gaa att      288
Leu Ile Thr Glu Gln Ala Ser Phe Thr Leu Pro Arg Ser Ala Glu Ile
                85                  90                  95

gaa ggc aaa tgt gca aac acc gag tcg gag atc cat atc acc tgg aag      336
Glu Gly Lys Cys Ala Asn Thr Glu Ser Glu Ile His Ile Thr Trp Lys
            100                 105                 110

aat aaa gcc tac aat ctg cga atc ttc ttc tct aag gag gtt cgt gaa      384
Asn Lys Ala Tyr Asn Leu Arg Ile Phe Phe Ser Lys Glu Val Arg Glu
        115                 120                 125

aaa agc acc gag gtg tgg aag atc agc aag gtc cag ttc atc tac gac      432
Lys Ser Thr Glu Val Trp Lys Ile Ser Lys Val Gln Phe Ile Tyr Asp
    130                 135                 140

acg tcg gag aat gcc acc aca tgg agc tca gcg ctg aga ttt tgg ctg      480
Thr Ser Glu Asn Ala Thr Thr Trp Ser Ser Ala Leu Arg Phe Trp Leu
145                 150                 155                 160

cac tat gct tca ctg cac ttg ctc tat agg gta cat tat agc gcc att      528
His Tyr Ala Ser Leu His Leu Leu Tyr Arg Val His Tyr Ser Ala Ile
                165                 170                 175

gat agg gag aga aac cca ggt cta ccc aca cac act gcg gag tgt att      576
Asp Arg Glu Arg Asn Pro Gly Leu Pro Thr His Thr Ala Glu Cys Ile
            180                 185                 190

ttt gtg act gta aat gca ttt cat cac tca aac aca cgt gac gaa cct      624
Phe Val Thr Val Asn Ala Phe His His Ser Asn Thr Arg Asp Glu Pro
        195                 200                 205

gta cgt gtg aaa tat atg atg tca ttc tcc caa acc agc atg ctg act      672
Val Arg Val Lys Tyr Met Met Ser Phe Ser Gln Thr Ser Met Leu Thr
    210                 215                 220

ctt gct ttt cct ctt tac gca gcc tac aag tgt atc acc gat cag cgg      720

Leu Ala Phe Pro Leu Tyr Ala Ala Tyr Lys Cys Ile Thr Asp Gln Arg
225                 230                 235                 240

gaa cgc cta gaa gag acc ctc ccc ctc atc ctg ggc ttc att ctg gcc       768
Glu Arg Leu Glu Glu Thr Leu Pro Leu Ile Leu Gly Phe Ile Leu Ala
                245                 250                 255

ctc atc atc gtc atc aca ctc aca gtc tac cac ttc cat ctg aaa atg       816
Leu Ile Ile Val Ile Thr Leu Thr Val Tyr His Phe His Leu Lys Met
            260                 265                 270

agc gcg agc cag ccc cag ctc ccc aga gac cgc tcc atg tac aag aac       864
Ser Ala Ser Gln Pro Gln Leu Pro Arg Asp Arg Ser Met Tyr Lys Asn
        275                 280                 285

atg tag                                                               870
Met

<210>  20
<211>  289
<212>  PRT
<213>  Tetraodon nigroviridis

<400>  20

Met Gly Arg Leu Ala Leu Gly Thr Thr Glu Ser Thr Lys Cys Leu Pro
1               5                   10                  15

Leu Ala Val Phe Gly Val Leu Ala Leu Ser Leu Val Leu Ala Glu Gln
            20                  25                  30

Glu Gly Glu Asn Leu Ser Gly Leu Ser Asn Asn Pro Asp Lys Ala Ile
        35                  40                  45

Phe Ala Val Arg Glu Asn Gly Thr Thr Cys Leu Met Val Glu Phe Ala
    50                  55                  60

Val Lys Phe Leu Val Pro Tyr Asp Val Leu Ala Leu Asn Gly Ile Asp
65                  70                  75                  80

Leu Ile Thr Glu Gln Ala Ser Phe Thr Leu Pro Arg Ser Ala Glu Ile
                85                  90                  95

Glu Gly Lys Cys Ala Asn Thr Glu Ser Glu Ile His Ile Thr Trp Lys
            100                 105                 110

Asn Lys Ala Tyr Asn Leu Arg Ile Phe Phe Ser Lys Glu Val Arg Glu
        115                 120                 125

Lys Ser Thr Glu Val Trp Lys Ile Ser Lys Val Gln Phe Ile Tyr Asp
    130                 135                 140

Thr Ser Glu Asn Ala Thr Thr Trp Ser Ser Ala Leu Arg Phe Trp Leu
145                 150                 155                 160

His Tyr Ala Ser Leu His Leu Leu Tyr Arg Val His Tyr Ser Ala Ile
                165                 170                 175

Asp Arg Glu Arg Asn Pro Gly Leu Pro Thr His Thr Ala Glu Cys Ile
            180                 185                 190

Phe Val Thr Val Asn Ala Phe His His Ser Asn Thr Arg Asp Glu Pro
        195                 200                 205

Val Arg Val Lys Tyr Met Met Ser Phe Ser Gln Thr Ser Met Leu Thr
    210                 215                 220

Leu Ala Phe Pro Leu Tyr Ala Ala Tyr Lys Cys Ile Thr Asp Gln Arg
225                 230                 235                 240

Glu Arg Leu Glu Glu Thr Leu Pro Leu Ile Leu Gly Phe Ile Leu Ala
                245                 250                 255

Leu Ile Ile Val Ile Thr Leu Thr Val Tyr His Phe His Leu Lys Met
            260                 265                 270

Ser Ala Ser Gln Pro Gln Leu Pro Arg Asp Arg Ser Met Tyr Lys Asn
        275                 280                 285

Met

<210>  21
<211>  885
<212>  DNA
<213>  Takifugu rubripes

<220>
<221>  CDS
<222>  (1)..(885)

<400>  21
atg gga cgg ctt ggt ttt ggc gtc cga ctt ttg ccg ctt gcc gtg ttt      48
Met Gly Arg Leu Gly Phe Gly Val Arg Leu Leu Pro Leu Ala Val Phe
1               5                   10                  15

ggt gtt ctg gcg ttg tcc ctg gtg ctg gcg gag cag gag ggc gag aac      96
Gly Val Leu Ala Leu Ser Leu Val Leu Ala Glu Gln Glu Gly Glu Asn
            20                  25                  30

ctg tcc gga ctg tcc aac aac ccg gac aaa gcc atc ttc gcg gtg cgg 144
Leu Ser Gly Leu Ser Asn Asn Pro Asp Lys Ala Ile Phe Ala Val Arg
        35                  40                  45

gag aac ggc acc aca tgt ctg atg gtg gag ttc gcc gtg aga ttc ctc 192
Glu Asn Gly Thr Thr Cys Leu Met Val Glu Phe Ala Val Arg Phe Leu
    50                  55                  60

gta cca tat gac gtg ctt gct ctc aat ggg ata gat ctt atc aca gaa 240
Val Pro Tyr Asp Val Leu Ala Leu Asn Gly Ile Asp Leu Ile Thr Glu
65                  70                  75                  80

cag gcg tca ttt acg ctg cct cgg agt gca gaa att gaa ggc aac tgc 288
Gln Ala Ser Phe Thr Leu Pro Arg Ser Ala Glu Ile Glu Gly Asn Cys
                85                  90                  95

gac acc agc gag tca gag atc cac atc acc tgg aag aat aaa gcc tac 336
Asp Thr Ser Glu Ser Glu Ile His Ile Thr Trp Lys Asn Lys Ala Tyr
            100                 105                 110

agt ctg cgc atc ttc ttc tct aag gag ttt cct gac aaa ggc acc gag 384
Ser Leu Arg Ile Phe Phe Ser Lys Glu Phe Pro Asp Lys Gly Thr Glu
        115                 120                 125

gtg tgg aag att agc aag gtc cag ttc atc tac gac acc tcg gag acg 432
Val Trp Lys Ile Ser Lys Val Gln Phe Ile Tyr Asp Thr Ser Glu Thr
    130                 135                 140

tct cat ttc atc aac gca tac aac ccc gct gta aac ggg ctg cgc tat 480
Ser His Phe Ile Asn Ala Tyr Asn Pro Ala Val Asn Gly Leu Arg Tyr
145                 150                 155                 160

gct tca ctg cac ttc ctc tat agg gta cat tat agc acc att cac agg 528
Ala Ser Leu His Phe Leu Tyr Arg Val His Tyr Ser Thr Ile His Arg
                165                 170                 175

gag aaa aaa caa ctt tta acc aca tac tgt gaa gca tat tgt tgt tac 576
Glu Lys Lys Gln Leu Leu Thr Thr Tyr Cys Glu Ala Tyr Cys Cys Tyr
            180                 185                 190

tgt aga tta gct cca tcg ctc aaa cac gtg ata aac att gaa ttg gtt 624
Cys Arg Leu Ala Pro Ser Leu Lys His Val Ile Asn Ile Glu Leu Val
        195                 200                 205

caa gag ttg acc gag ctg ctg cat tct aaa cag atg atg tca ctc tct 672
Gln Glu Leu Thr Glu Leu Leu His Ser Lys Gln Met Met Ser Leu Ser
    210                 215                 220

ccc ctg att atg ctg act ctt ctg cct cct tac aca gcc tac aag tgt 720
Pro Leu Ile Met Leu Thr Leu Leu Pro Pro Tyr Thr Ala Tyr Lys Cys
225                 230                 235                 240

atc act gat cag cgg gag cgc ctg gaa gag acc ctc ccg ctc atc ctg 768
Ile Thr Asp Gln Arg Glu Arg Leu Glu Glu Thr Leu Pro Leu Ile Leu
                245                 250                 255

ggc ttc att cta gcc ctc atc att gta atc aca ctg acc gtc tat cac 816
Gly Phe Ile Leu Ala Leu Ile Ile Val Ile Thr Leu Thr Val Tyr His
            260                 265                 270

ttt cac cta aaa atg aca gcg agc cag ccc cag ctc ccc aga gac cgc 864

Phe His Leu Lys Met Thr Ala Ser Gln Pro Gln Leu Pro Arg Asp Arg
        275                 280                 285

tcc atg tac aag aac atg taa 885
Ser Met Tyr Lys Asn Met
    290

<210> 22
<211> 294
<212> PRT
<213> Takifugu rubripes

<400> 22

Met Gly Arg Leu Gly Phe Gly Val Arg Leu Leu Pro Leu Ala Val Phe
1               5                   10                  15

Gly Val Leu Ala Leu Ser Leu Val Leu Ala Glu Gln Glu Gly Glu Asn
            20                  25                  30

Leu Ser Gly Leu Ser Asn Asn Pro Asp Lys Ala Ile Phe Ala Val Arg
        35                  40                  45

Glu Asn Gly Thr Thr Cys Leu Met Val Glu Phe Ala Val Arg Phe Leu
    50                  55                  60

Val Pro Tyr Asp Val Leu Ala Leu Asn Gly Ile Asp Leu Ile Thr Glu
65                  70                  75                  80

Gln Ala Ser Phe Thr Leu Pro Arg Ser Ala Glu Ile Glu Gly Asn Cys
                85                  90                  95

Asp Thr Ser Glu Ser Glu Ile His Ile Thr Trp Lys Asn Lys Ala Tyr
            100                 105                 110

Ser Leu Arg Ile Phe Phe Ser Lys Glu Phe Pro Asp Lys Gly Thr Glu
        115                 120                 125

Val Trp Lys Ile Ser Lys Val Gln Phe Ile Tyr Asp Thr Ser Glu Thr
    130                 135                 140

Ser His Phe Ile Asn Ala Tyr Asn Pro Ala Val Asn Gly Leu Arg Tyr
145                 150                 155                 160

Ala Ser Leu His Phe Leu Tyr Arg Val His Tyr Ser Thr Ile His Arg
                165                 170                 175

Glu Lys Lys Gln Leu Leu Thr Thr Tyr Cys Glu Ala Tyr Cys Cys Tyr
            180                 185                 190

Cys Arg Leu Ala Pro Ser Leu Lys His Val Ile Asn Ile Glu Leu Val
        195                 200                 205

Gln Glu Leu Thr Glu Leu Leu His Ser Lys Gln Met Met Ser Leu Ser
    210                 215                 220

Pro Leu Ile Met Leu Thr Leu Leu Pro Pro Tyr Thr Ala Tyr Lys Cys
225                 230                 235                 240

Ile Thr Asp Gln Arg Glu Arg Leu Glu Glu Thr Leu Pro Leu Ile Leu
                245                 250                 255

Gly Phe Ile Leu Ala Leu Ile Ile Val Ile Thr Leu Thr Val Tyr His
            260                 265                 270

Phe His Leu Lys Met Thr Ala Ser Gln Pro Gln Leu Pro Arg Asp Arg
        275                 280                 285

Ser Met Tyr Lys Asn Met
    290

<210>  23
<211>  888
<212>  DNA
<213>  Drosophila melanogaster

<220>
<221>  CDS
<222>  (1)..(888)

<400>  23
atg atg aaa ctg tcc ggt ctt agg cgg tcc tta ccc agg gac caa gga        48
Met Met Lys Leu Ser Gly Leu Arg Arg Ser Leu Pro Arg Asp Gln Gly
1               5                   10                  15

ttg ggc aac tgc ctc aac gtg gca ctg att tac tca gcg ctc cta gca        96
Leu Gly Asn Cys Leu Asn Val Ala Leu Ile Tyr Ser Ala Leu Leu Ala
            20                  25                  30

ctt ttc atg acc gcc gcc cag gga cta cag ctg agt gcc ttc aca acg       144
Leu Phe Met Thr Ala Ala Gln Gly Leu Gln Leu Ser Ala Phe Thr Thr
        35                  40                  45

aag acg ccg cgg gtc agc aag tac aca acc aag tcg cca tcg gcc tcc       192
Lys Thr Pro Arg Val Ser Lys Tyr Thr Thr Lys Ser Pro Ser Ala Ser
    50                  55                  60

tcc atg aac cac gat gcc acc gcc tcg ctg tat cgt ttc aat gcc acc       240
Ser Met Asn His Asp Ala Thr Ala Ser Leu Tyr Arg Phe Asn Ala Thr
65                  70                  75                  80

aac ggc atc acc tgc att ctg atg cag gtg gac gga ctt atc agc att 288
Asn Gly Ile Thr Cys Ile Leu Met Gln Val Asp Gly Leu Ile Ser Ile
                85                  90                  95

aag tac cgg aac aag ctg aac gag gat gtg gag gcc gat ttg tac atg 336
Lys Tyr Arg Asn Lys Leu Asn Glu Asp Val Glu Ala Asp Leu Tyr Met
            100                 105                 110

ccg gat gat ccc cag ctg agt ggc gag tgc gtc gag tcc aac atg gaa 384
Pro Asp Asp Pro Gln Leu Ser Gly Glu Cys Val Glu Ser Asn Met Glu
        115                 120                 125

att ctc acc atg gac ttt aag ggt ttc cgg ctg tcc atg aca ttt aaa 432
Ile Leu Thr Met Asp Phe Lys Gly Phe Arg Leu Ser Met Thr Phe Lys
    130                 135                 140

aag tcg tcc ggc ggc gag ggt tgg tat atc aac ctg ttt gaa ctg acc 480
Lys Ser Ser Gly Gly Glu Gly Trp Tyr Ile Asn Leu Phe Glu Leu Thr
145                 150                 155                 160

tac tcg tcc tcc aac tgg ctg ttc gag cac ccc gat cgt ccc aac ttg 528
Tyr Ser Ser Ser Asn Trp Leu Phe Glu His Pro Asp Arg Pro Asn Leu
                165                 170                 175

gac gtg agg ctg acc tcg ccc gcc cag acg ccg atg tac ttc ccc acg 576
Asp Val Arg Leu Thr Ser Pro Ala Gln Thr Pro Met Tyr Phe Pro Thr
            180                 185                 190

ccg gtg ggt aag tcc tat gtc tgc gac aag gag cag act gtg atc atg 624
Pro Val Gly Lys Ser Tyr Val Cys Asp Lys Glu Gln Thr Val Ile Met
        195                 200                 205

tac gcc ccg cac gat tcc ggc gac ctg tcg ggg cat ata gcc aag ctg 672
Tyr Ala Pro His Asp Ser Gly Asp Leu Ser Gly His Ile Ala Lys Leu
    210                 215                 220

tat ctg cgc gac atg cac atg cag agc ttc atg ttc aag gac agc ggc 720
Tyr Leu Arg Asp Met His Met Gln Ser Phe Met Phe Lys Asp Ser Gly
225                 230                 235                 240

aag tgg ggc ccg tcc ttc cac tgc agc gcc aca gga tcc tat cgc gac 768
Lys Trp Gly Pro Ser Phe His Cys Ser Ala Thr Gly Ser Tyr Arg Asp
                245                 250                 255

gag aca gcg ccc ctg gcc gtg gga act gca ctg gcc att gcc gtg cta 816
Glu Thr Ala Pro Leu Ala Val Gly Thr Ala Leu Ala Ile Ala Val Leu
            260                 265                 270

ctg acc ata tcc gga tac ggc ggt tgg aga tac ttc aaa atc aag aag 864
Leu Thr Ile Ser Gly Tyr Gly Gly Trp Arg Tyr Phe Lys Ile Lys Lys
        275                 280                 285

gtt cag tac ggt tct atg gag tga 888
Val Gln Tyr Gly Ser Met Glu
    290                 295

<210> 24
<211> 295
<212> PRT
<213> Drosophila melanogaster

<400> 24

Met Met Lys Leu Ser Gly Leu Arg Arg Ser Leu Pro Arg Asp Gln Gly
1               5                   10                  15

Leu Gly Asn Cys Leu Asn Val Ala Leu Ile Tyr Ser Ala Leu Leu Ala
            20                  25                  30

Leu Phe Met Thr Ala Ala Gln Gly Leu Gln Leu Ser Ala Phe Thr Thr
        35                  40                  45

Lys Thr Pro Arg Val Ser Lys Tyr Thr Thr Lys Ser Pro Ser Ala Ser
    50                  55                  60

Ser Met Asn His Asp Ala Thr Ala Ser Leu Tyr Arg Phe Asn Ala Thr
65                  70                  75                  80

Asn Gly Ile Thr Cys Ile Leu Met Gln Val Asp Gly Leu Ile Ser Ile
                85                  90                  95

Lys Tyr Arg Asn Lys Leu Asn Glu Asp Val Glu Ala Asp Leu Tyr Met
            100                 105                 110

Pro Asp Asp Pro Gln Leu Ser Gly Glu Cys Val Glu Ser Asn Met Glu
        115                 120                 125

Ile Leu Thr Met Asp Phe Lys Gly Phe Arg Leu Ser Met Thr Phe Lys
    130                 135                 140

Lys Ser Ser Gly Gly Glu Gly Trp Tyr Ile Asn Leu Phe Glu Leu Thr
145                 150                 155                 160

Tyr Ser Ser Ser Asn Trp Leu Phe Glu His Pro Asp Arg Pro Asn Leu
                165                 170                 175

Asp Val Arg Leu Thr Ser Pro Ala Gln Thr Pro Met Tyr Phe Pro Thr
            180                 185                 190

Pro Val Gly Lys Ser Tyr Val Cys Asp Lys Glu Gln Thr Val Ile Met
        195                 200                 205

Tyr Ala Pro His Asp Ser Gly Asp Leu Ser Gly His Ile Ala Lys Leu
    210                 215                 220

Tyr Leu Arg Asp Met His Met Gln Ser Phe Met Phe Lys Asp Ser Gly
225                 230                 235                 240

Lys Trp Gly Pro Ser Phe His Cys Ser Ala Thr Gly Ser Tyr Arg Asp
                245                 250                 255

Glu Thr Ala Pro Leu Ala Val Gly Thr Ala Leu Ala Ile Ala Val Leu
            260                 265                 270

Leu Thr Ile Ser Gly Tyr Gly Gly Trp Arg Tyr Phe Lys Ile Lys Lys
        275                 280                 285

Val Gln Tyr Gly Ser Met Glu
    290                 295

<210>  25
<211>  280
<212>  PRT
<213>  Artificial sequence

<220>
<223>  Mouse BAD-LAMP Y276A mutant

<400>  25

Met Asp Leu Arg Val Arg Thr Leu Leu Gly Gly Asp Arg Leu Arg Ile
1               5                   10                  15

Leu Leu Met Phe Phe His Val Met Val Gln Thr Val Ala Glu Gln Glu
            20                  25                  30

Val Glu Asn Leu Ser Gly Leu Ser Thr Asn Pro Glu Lys Asp Ile Phe
        35                  40                  45

Val Val Arg Glu Asn Gly Thr Thr Cys Leu Met Ala Glu Phe Ala Ala
    50                  55                  60

Lys Phe Ile Val Pro Tyr Asp Val Trp Ala Ser Asn Tyr Val Asp Leu
65                  70                  75                  80

Ile Thr Glu Gln Ala Glu Ile Ser Leu Thr Arg Gly Ala Glu Val Lys
                85                  90                  95

Gly His Cys Gly His Asn Glu Ser Glu Leu Glu Val Phe Trp Val Asp
            100                 105                 110

His Ala Tyr Thr Leu Arg Met Leu Phe Val Lys Glu Ser His Asn Thr
        115                 120                 125

Ser Lys Gly Pro Glu Ala Thr Trp Asn Leu Asn Lys Val His Phe Val

    130                 135                 140

Tyr Asp Ser Ser Glu Lys Thr His Phe Lys Ala Pro Val Lys Val Asn
145                 150                 155                 160

Lys Tyr Ile Ala Ser Ser His His Leu Ser Ala Leu Val Thr Pro Ala
                165                 170                 175

Gly Met Ser Tyr Glu Cys Gln Ala Gln Gln Thr Ile Ser Leu Ala Ser
            180                 185                 190

Ser Asp Pro Gln Lys Thr Val Thr Met Ile Leu Ser Ala Val His Ile
        195                 200                 205

Gln Pro Phe Asp Ile Ile Ser Asp Phe Val Phe Ser Glu Glu His Lys
    210                 215                 220

Cys Pro Val Asp Glu Gln Glu Gln Leu Glu Glu Thr Leu Pro Leu Ile
225                 230                 235                 240

Leu Gly Leu Ile Leu Gly Leu Val Ile Val Ile Thr Leu Val Ile Tyr
                245                 250                 255

His Ile His His Lys Met Thr Ala Asn Gln Val Gln Ile Pro Arg Asp
            260                 265                 270

Arg Ser Gln Ala Lys His Met Gly
        275                 280

<210> 26
<211> 16
<212> PRT
<213> Artificial sequence

<220>
<223> BAD-LAMP fragment

<400> 26

Lys Met Thr Ala Asn Gln Val Gln Ile Pro Arg Asp Arg Ser Gln Cys
1               5                   10                  15

<210> 27
<211> 14
<212> PRT
<213> Artificial sequence

<220>
<223> BAD-LAMP fragment

<400>  27

Lys Gln Ile Pro Arg Asp Arg Ser Gln Tyr Lys His Met Cys
1               5                   10


<210>  28
<211>  20
<212>  PRT
<213>  Artificial sequence

<220>
<223>  BAD-LAMP fragment

<400>  28

Lys Met Thr Ala Asn Gln Val Gln Ile Pro Arg Asp Arg Ser Gln Tyr
1               5                   10                  15


Lys His Met Cys
            20


<210>  29
<211>  18
<212>  DNA
<213>  Artificial sequence

<220>
<223>  PCR primer

<400>  29
accggccact ttgaggga                                                    18


<210>  30
<211>  18
<212>  DNA
<213>  Artificial sequence

<220>
<223>  PCR primer

<400>  30
ggggcggcct ttgcagca                                                    18


<210>  31
<211>  236
<212>  PRT
<213>  Artificial sequence

<220>
<223>  BAD-LAMP fragment

<400>  31

Met Asp Leu Gln Gly Arg Gly Val Pro Ser Ile Asp Arg Leu Arg Val
1               5                   10                  15

Leu Leu Met Leu Phe His Thr Met Ala Gln Ile Met Ala Glu Gln Glu
            20                  25                  30

Val Glu Asn Leu Ser Gly Leu Ser Thr Asn Pro Glu Lys Asp Ile Phe
        35                  40                  45

Val Val Arg Glu Asn Gly Thr Thr Cys Leu Met Ala Glu Phe Ala Ala
    50                  55                  60

Lys Phe Ile Val Pro Tyr Asp Val Trp Ala Ser Asn Tyr Val Asp Leu
65                  70                  75                  80

Ile Thr Glu Gln Ala Asp Ile Ala Leu Thr Arg Gly Ala Glu Val Lys
                85                  90                  95

Gly Arg Cys Gly His Ser Gln Ser Glu Leu Gln Val Phe Trp Val Asp
            100                 105                 110

Arg Ala Tyr Ala Leu Lys Met Leu Phe Val Lys Glu Ser His Asn Met
        115                 120                 125

Ser Lys Gly Pro Glu Ala Thr Trp Arg Leu Ser Lys Val Gln Phe Val
    130                 135                 140

Tyr Asp Ser Ser Glu Lys Thr His Phe Lys Asp Ala Val Ser Ala Gly
145                 150                 155                 160

Lys His Thr Ala Asn Ser His His Leu Ser Ala Leu Val Thr Pro Ala
                165                 170                 175

Gly Lys Ser Tyr Glu Cys Gln Ala Gln Gln Thr Ile Ser Leu Ala Ser
            180                 185                 190

Ser Asp Pro Gln Lys Thr Val Thr Met Ile Leu Ser Ala Val His Ile
        195                 200                 205

Gln Pro Phe Asp Ile Ile Ser Asp Phe Val Phe Ser Glu Glu His Lys
    210                 215                 220

Cys Pro Val Asp Glu Arg Glu Gln Leu Glu Glu Thr
225                 230                 235